# EUROPÄISCHE PATENTSCHRIFT

(11) **EP 1 731 135 B1**
(45) Veröffentlichungstag und Bekanntmachung des Hinweises auf die Patenterteilung: **24.09.2008**
(21) Anmeldenummer: 06011337.0
(22) Anmeldetag: 01.06.2006
(51) Int. Cl.: A61K 8/81, A61K 8/92, A61K 31/75, A61Q 1/10, A61Q 9/04, A61Q 11/00, A61Q 15/00, A61Q 19/00

(54) **Kosmetische, pharmazeutische und dermatologische Zubereitungen enthaltend Homo- und/oder Copolymerwachse aus den Monomeren Ethylen und/oder Propylen**
Cosmetic, pharmaceutical and dermatological compositions containing homo- and/or copolymer waxes of the monomers ethylene and/or propylene
Compositions cosmétiques, pharmaceutiques et dermatologiques contenant des cires de homo- et/ou copolymères formée par les monomères éthylene et/ou propylene

(30) Priorität: 08.06.2005 DE 102005026278
(43) Veröffentlichungstag der Anmeldung: 13.12.2006
(73) Patentinhaber: Clariant Produkte (Deutschland) GmbH, 65929 Frankfurt am Main (DE)
(72) Erfinder: Lukasch, Anton, Dipl.-Ing., 86405 Meitingen (DE); Hohner, Gerd, Dr., 86368 Gersthofen (DE); Michaelis, Heike, 64295 Darmstadt (DE); Lachmann, Angela, Dr., 65779 Kelkheim-Fischbach (DE); Herrmann, Hans-Friedrich, Dr., 64521 Gross-Gerau (DE)
(74) Vertreter: Paczkowski, Marcus

(56) Entgegenhaltungen:
- EP-A- 0 571 882
- WO-A-02/49592
- DE-A1- 10 064 799

## Beschreibung

Die Erfindung betrifft kosmetische, pharmazeutische und dermatologische Zubereitungen enthaltend Homo- und/oder Copolymerwachse, erhalten durch Niederdruckpolymerisation von Ethylen und/oder Propylen in Gegenwart eines Metallocenkatalysators.

Wachse und wachsähnliche Substanzen bestimmen hauptsächlich die Konsistenz von vielen Kosmetik-Produkten. Wachse werden verwendet, um Härte und Festigkeit von kosmetischen Produkten zu beeinflussen. Je mehr besonders harte und erst bei hohen Temperaturen schmelzende Wachse eingesetzt werden, umso fester wird das Produkt. Wachse werden auch in flüssigen, cremeförmigen und gelartigen Zubereitungen vorteilhaft eingesetzt. Sie verbessern die Wasserfestigkeit und die Köhasion der Mittel auf Haut und Haaren, sind geruchs- und geschmacksneutral und gut verarbeitbar.

In der Kosmetik werden häufig natürliche Wachse tierischen und pflanzlichen Ursprungs, wie Bienenwachs, Beerenwachs, Rosenwachs, Japanwachs, Chinawachs, Schellackwachs, Quittenwachs, Shea-Butter, Candelillawachs, Carnaubawachs, Lanolin (Wollfett), Jojobaöl und Jojobawachs verwendet. Wegen einer Belastung natürlicher Wachse durch Pestizide sucht man nach alternativen, synthetisch hergestellten Wachsen, die frei von Pflanzenschutzmitteln und allergenen Stoffen sind. Darüberhinaus sucht man nach wachsartigen Substanzen, die eine gute Hautsensorik zeigen, eine gute Filmbildung und -stabilität aufweisen und mit üblichen kosmetischen Inhaltsstoffen kompatibel sind.

In DE 100 64 799 wird die Verwendung von Ethylenpolymerwachsen und Ethylencopolymerwachsen, hergestellt unter Hochdruckbedingungen, in kosmetischen Zubereitungen beschrieben. Bei diesem Verfahren werden aliphatische und alicyclische Ketone als Molekulargewichtsregler eingesetzt, um Ethylen(co)polymerwachse mit geeigneten Molekulargewichten zu erhalten. Ketone können sensibilisierend wirken und sind in kosmetischen Formulierungen unerwünscht.

In WO 2002/049592 werden Antiperspirantien und Deodorantien beschrieben, die neben Cyclomethiconen und Wirkstoffen niedermolekulare, überwiegend lineare Polyethylenwachse mit Molekulargewichten von 300 bis 3000 g/mol, bevorzugt 300 bis 500 g/mol, beispielsweise Performalene^{®}, enthalten. Diese niedermolekularen Polyethylenwachse sind spröde, wenig elastisch und haben eine geringe Haftung auf Haut und Haaren.

Es bestand die Aufgabe, Substanzen für kosmetische, pharmazeutische und dermatologische Zubereitungen zur Verfügung zu stellen, die ähnlich gute konsistenzgebende Eigenschaften wie natürlich vorkommende Wachse aufweisen, mit wässrigen Systemen und mit Ölsystemen gut verträglich sind, ein klares visuelles Erscheinungsbild haben, leicht verarbeitbar und kompatibel mit Wirksubstanzen (z.B. Sonnenschutzfiltern) sind, aber auch hautverträglich und toxikologisch unbedenklich sind, eine gute Hautsensorik aufweisen, auf Haut und Haaren gut haften und wasserfest sind.

Es wurde überraschend gefunden, dass diese Aufgabe gelöst wird durch bestimmte Homo- und/oder Copolymerwachse aus den Monomeren Ethylen und/oder Propylen mit einem gewichtsmittleren Molekulargewicht Mw von kleiner oder gleich 25000 g/mol und enger Molekulargewichtsverteilung, hergestellt durch Niederdruckpolymerisation von Ethylen und/oder Propylen in Gegenwart eines Metallocenkatalysators.

Gegenstand der Erfindung sind kosmetische, pharmazeutische und dermatologische Zubereitungen enthaltend ein oder mehrere Homo- und/oder Copolymerwachse aus den Monomeren Ethylen und/oder Propylen, dadurch gekennzeichnet, dass die Homo- und Copolymerwachse ein gewichtsmittleres Molekulargewicht Mw von kleiner oder gleich 25000 g/mol, ein zahlenmittleres Molekulargewicht Mn von kleiner oder gleich 15000 g/mol, eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5 besitzen und durch Metallocenkatalyse erhalten worden sind und wobei die Copolymerwachse, bezogen auf das Gesamtgewicht der Copolymerwachse, 0,1 bis 30,0 Gew.-% an Struktureinheiten hervorgegangen aus dem einen Monomer und 70,0 bis 99,9 Gew.-% an Struktureinheiten hervorgegangen aus dem anderen Monomer enthalten.

Diese Homo- und/oder Copolymerwachse haben ein weißes Aussehen, sind geruchs- und geschmacksneutral, gut verarbeitbar und gut geeignet zur FormStabilisierung fester Mittel, beispielsweise von Sticks. Sie sind geeignet zur Viskositätseinstellung cremiger Emulsionen oder Dispersionen, sowie hydroxysäurehaltiger und elektrolythaltiger Mittel und können zu fließfähigen Zubereitungen verarbeitet werden. Sie verbessern signifikant das Aufnahmevermögen von Pigmenten in der Lipidphase und die Pigmentdispergierung, sowie die Wirkung von Effektpigmenten. Sehr vorteilhaft für die kosmetische Anwendung ist die Wasserfestigkeit und Haftfestigkeit der Mittel. Die Migration fester Inhaltsstoffe (z.B. Pigmente) wird unterdrückt, ebenso die Tendenz einzelner Inhaltsstoffe in die Haut zu penetrieren. Damit wird eine Reduzierung der Reizwirkung von Inhaltsstoffen erreicht. Vorteilhafterweise können die Homo-und/oder Copolymerwachse als Wirkstoffträgermatrix für eine gezielte und verzögerte Freisetzung von Wirksubstanzen eingesetzt werden.

Die Homo- und Copolymerwachse aus Ethylen und/oder Propylen können ebenfalls vorteilhafterweise in mikronisierter Form in Peelings und Zahnpasten als schonende Abrasivkomponente eingesetzt werden.

Mit den Homo- und Copolymerwachsen, hergestellt mit Metallocenkatalysatoren lassen sich auch sehr feste Pasten und Sticks herstellen mit sehr guter Lösemittelbindung, insbesondere von aliphatischen Lösemitteln.

In einer bevorzugten Ausführungsform enthalten die erfindungsgemäßen kosmetischen, pharmazeutischen oder dermatologischen Zubereitungen ein oder mehrere Homopolymerwachse aus dem Monomer Ethylen, dadurch gekennzeichnet, dass die Homopolymerwachse ein gewichtsmittleres Molekulargewicht Mw von kleiner oder gleich 25000 g/mol, vorzugsweise von 1000 bis 22000 g/mol und besonders bevorzugt von 4000 bis 20000 g/mol, ein zahlenmittleres Molekulargewicht Mn von kleiner oder gleich 15000 g/mol, vorzugsweise von 500 bis 12000 g/mol und besonders bevorzugt von 1000 bis 5000 g/mol und eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5 besitzen und durch Metallocenkatalyse erhalten worden sind.

In einer weiteren bevorzugten Ausführungsform enthalten die erfindungsgemäßen kosmetischen, pharmazeutischen oder dermatologischen Zubereitungen ein oder mehrere Copolymerwachse aus den Monomeren Ethylen und Propylen, dadurch gekennzeichnet, dass die Copolymerwachse ein gewichtsmittleres Molekulargewicht Mw von kleiner oder gleich 25000 g/mol, vorzugsweise von 2000 bis 22000 g/mol und besonders bevorzugt von 4000 bis 20000 g/mol, ein zahlenmittleres Molekulargewicht Mn von kleiner oder gleich 15000 g/mol, vorzugsweise von 1000 bis 12000 g/mol und besonders bevorzugt von 2000 bis 10000 g/mol, eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5 besitzen und durch Metallocenkatalyse erhalten worden sind und wobei die Copolymerwachse, bezogen auf das Gesamtgewicht der Copolymerwachse, 70,0 bis 99,9 Gew.-% an Struktureinheiten hervorgegangen aus dem Monomer Ethylen und 0,1 bis 30,0 Gew.-% an Struktureinheiten hervorgegangen aus dem Monomer Propylen enthalten.

In einer weiteren bevorzugten Ausführungsform enthalten die erfindungsgemäßen kosmetischen, pharmazeutischen oder dermatologischen Zubereitungen ein oder mehrere Homopolymerwachse aus dem Monomer Propylen, dadurch gekennzeichnet, dass die Homopolymerwachse ein gewichtsmittleres Molekulargewicht Mw von kleiner oder gleich 25000 g/mol, vorzugsweise von 2000 bis 22000 g/mol und besonders bevorzugt von 4000 bis 22000 g/mol, ein zahlenmittleres Molekulargewicht Mn von kleiner oder gleich 15000 g/mol, vorzugsweise von 1000 bis 12000 g/mol und besonders bevorzugt von 2000 bis 10000 g/mol, eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5 sowie einen isotaktischen Index von größer als 70 %, vorzugsweise von 75 bis 95 % und besonders bevorzugt von 80 bis 90 % besitzen und durch Metallocenkatalyse erhalten worden sind.

In einer weiteren bevorzugten Ausführungsform enthalten die erfindungsgemäßen kosmetischen, pharmazeutischen oder dermatologischen Zubereitungen ein oder mehrere Copolymerwachse aus den Monomeren Ethylen und Propylen, dadurch gekennzeichnet, dass die Copolymerwachse ein gewichtsmittleres Molekulargewicht Mw von kleiner oder gleich 25000 g/mol, vorzugsweise von 2000 bis 22000 g/mol und besonders bevorzugt von 4000 bis 20000 g/mol, ein zahlenmittleres Molekulargewicht Mn von kleiner oder gleich 15000 g/mol, vorzugsweise von 1000 bis 12000 g/mol und besonders bevorzugt von 2000 bis 10000 g/mol, eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5 besitzen und durch Metallocenkatalyse erhalten worden sind und wobei die Copolymerwachse, bezogen auf das Gesamtgewicht der Copolymerwachse, 0,1 bis 30,0 Gew.-% an Struktureinheiten hervorgegangen aus dem Monomer Ethylen und 70,0 bis 99,9 Gew.-% an Struktureinheiten hervorgegangen aus dem Monomer Propylen enthalten.

In einer weiteren bevorzugten Ausführungsform enthalten die erfindungsgemäßen kosmetischen, pharmazeutischen oder dermatologischen Zubereitungen ein oder mehrere der oben beschriebenen Homo- und/oder Copolymerwachse aus den Monomeren Ethylen und/oder Propylen, dadurch gekennzeichnet, dass die Homo-und Copolymerwachse einen Tropf- oder Erweichungspunkt Ring/Kugel zwischen 80 und 165°C haben und eine Schmelzviskosität, gemessen bei einer Temperatur von 170°C, zwischen 20 und 40000 mPa.s besitzen.

In einer weiteren bevorzugten Ausführungsform enthalten die erfindungsgemäßen kosmetischen, pharmazeutischen oder dermatologischen Zubereitungen ein oder mehrere der oben beschriebenen Copolymerwachse aus den Monomeren Ethylen und Propylen, dadurch gekennzeichnet, daß diese eine Glasübergangstemperatur von maximal -20°C aufweisen.

In einer weiteren bevorzugten Ausführungsform enthalten die erfindungsgemäßen kosmetischen, pharmazeutischen oder dermatologischen Zubereitungen ein oder mehrere der oben beschriebenen Homo- und/oder Copolymerwachse aus den Monomeren Ethylen und/oder Propylen, dadurch gekennzeichnet, dass diese einen Kristallisationsgrad von mehr als 55 % und vorzugsweise zwischen 60 und 90 % aufweisen.

In einer weiteren bevorzugten Ausführungsform enthalten die erfindungsgemäßen kosmetischen, pharmazeutischen oder dermatologischen Zubereitungen ein oder mehrere der oben beschriebenen Homo- und/oder Copolymerwachse aus den Monomeren Ethylen und/oder Propylen, dadurch gekennzeichnet, dass diese einen Kristallisationsgrad unterhalb 55 % und vorzugsweise unterhalb von 50 % aufweisen.

Die erfindungsgemäß in den kosmetischen, pharmazeutischen oder dermatologischen Zubereitungen eingesetzten Homo- und Copolymerwachse aus den Monomeren Ethylen und/oder Propylen können z.B. nach dem in EP 571 882 beschriebenen Verfahren mit den dort genannten Metallocenkatalysatoren hergestellt werden. Geeignete Verfahren sind z.B. auch die Suspensions-, Lösungs- oder Gasphasenpolymerisation der Olefine in Gegenwart von Metallocenkatalysatoren, wobei auch in den Monomeren polymerisiert werden kann.

Unter den mittles Metallocen-Katalyse hergestellten Homo- und/oder Copolymerwachsen werden im Rahmen der vorliegenden Erfindung sowohl unmodifizierte als auch polar modifizierte Homo- und/oder Copolymerwachse verstanden. In den erfindungsgemäßen kosmetischen, pharmazeutischen oder dermatologischen Zubereitungen können das eine oder die mehreren Homo-und/oder Copolymerwachse entweder ausschließlich aus unmodifizierten oder ausschließlich aus polar modifizierten oder auch aus Mischungen aus unpolaren und polar modifizierten Homo- und/oder Copolymerwachsen ausgewählt sein.

Polar modifizierte Wachse können in bekannter Weise aus unmodifizierten Wachsen durch Oxidation mit sauerstoffhaltigen Gasen, beispielsweise Luft, oder durch Pfropfreaktion mit polaren Monomeren, beispielsweise Maleinsäure oder Acrylsäure oder Derivaten dieser Säuren hergestellt werden. Die polare Modifizierung von Metallocen-Polyolefinwachsen durch Oxidation mit Luft ist beispielsweise in EP 0 890 583 A1, die Modifizierung durch Pfropfung beispielsweise in US 5,998,547 beschrieben.

In einer bevorzugten Ausführungsform enthalten die erfindungsgemäßen kosmetischen, pharmazeutischen oder dermatologischen Zubereitungen ein oder mehrere mittels Metallocen-Katalyse hergestellte Homo- und/oder Copolymerwachse, die nicht polar modifiziert sind und zusätzlich ein oder mehrere mittels Metallocen-Katalyse hergestellte Homo- und/oder Copolymerwachse, die polar modifiziert sind.

In einer weiteren bevorzugten Ausführungsform sind die in den erfindungsgemäßen kosmetischen, pharmazeutischen oder dermatologischen Zubereitungen enthaltenen ein oder mehreren mittels Metallocen-Katalyse hergestellten Homo- und/oder Copolymerwachse aus nicht polar modifizierten Homo- und/oder Copolymerwachsen ausgewählt.

In einer weiteren bevorzugten Ausführungsform sind die in den erfindungsgemäßen kosmetischen, pharmazeutischen oder dermatologischen Zubereitungen enthaltenen ein oder mehreren mittels Metallocen-Katalyse hergestellten Homo- und/oder Copolymerwachse aus polar modifizierten Homo- und/oder Copolymerwachsen ausgewählt.

Polar modifizierte Homo- und/oder Copolymerwachse werden vorzugsweise z.B. dann verwendet, wenn es sich bei den erfindungsgemäßen kosmetischen, pharmazeutischen oder dermatologischen Zubereitungen um Emulsionen handelt.

Das Eigenschaftsprofil der erfindungsgemäß eingesetzten Homo- und Copolymerwachse wird durch die Wahl der Monomeren, durch deren Gewichtsverhältnisse, durch die mittleren Molekulargewichte Mw und Mn, durch die Molmassenverteilung Mw/Mn, durch die Taktizitäten im Falle der Propylenhomopolymerwachse und durch den Kristallisationsgrad der Homo- oder Copolymerwachse bestimmt.

Die Schmelzen sind klar und transparent. Die Tropf- oder Erweichungspunkte Ring/Kugel liegen im Bereich von 80 bis 165°C und vorzugsweise im Bereich von 85 bis 145°C und die Dichten im Bereich von 0,80 bis 1,00 g/cm³ und vorzugsweise im Bereich von 0,88 bis 0,98 g/cm³. Die Viskositäten für Ethylenhomo- oder -copolymerwachse, gemessen bei 170°C, liegen vorzugsweise im Bereich von 20 bis 10000 mPa.s und die Viskositäten für Propylenhomo- oder -copolymerwachse, gemessen bei 170°C, liegen vorzugsweise im Bereich von 50 bis 10000 mPa.s und besonders bevorzugt im Bereich von 60 bis 6000 mPa.s.

Die Nadelpenetrationswerte der Homo- und Coplymerwachse liegen im Bereich von kleiner als 1 bis 10 x 0,1 mm und vorzugsweise von 1 bis 5 x 0,1 mm.

Beim Auftragen von Formulierungen enthaltend diese Homo- und/oder Copolymerwachse auf die Haut oder auf die Haare entstehen wasserabweisende Filme, die die Migration und das Aus- und Abwaschen von Inhaltsstoffen der kosmetischen, pharmazeutischen und dermatologischen Mittel, insbesondere von Wirksubstanzen und/oder Pigmenten, verhindern.

Zudem zeigen die erfindungsgemäß eingesetzten Homo- und Copolymerwachse ein sehr gutes Lösemittelbindevermögen, insbesondere von aliphatischen Lösemitteln, beispielsweise von Benzinen, Weißölen und Paraffinöl und können mit der flüssigen Lipidphase zu festen, temperaturbeständigen Mitteln, beispielsweise Lippenstiften, verarbeitet werden.

Mit diesen oben beschriebenen geruchsneutralen weißen Homo- und Copolymerwachsen können zusammen mit Lösemitteln und Ölkomponenten streichfähige oder fließfähige Cremes, Cremeschäume oder Pasten gefertigt werden, welche bei 20°C eine Viskosität von mehr als 500 mPa.s aufweisen. Sie sind ausgezeichnete Konsistenzgeber, insbesondere für Formulierungen auf Öl-Basis und besitzen gute Absorptionseigenschaften, die für die Absorption von Ölen und die Dispersion von Pigmenten, Geruchsstoffen oder festen Wirkstoffen und anderen festen Zusatzstoffen genutzt werden können.

Sehr vorteilhaft können die Homo- und Copolymerwachse als Absrasivkomponente, bevorzugt in Zahnpflegemittel und Peelings eingesetzt werden.

Aufgrund ihrer Härte verleihen die erfindungsgemäß eingesetzten Homo- und/oder Copolymerwachse z.B. Lippenstiften, Kajal- und Mascarastiften Stabilität auch bei höheren Temperaturen. Als Binder kann das Wachs zusammen mit Lanolin, Paraffinöl, Isopropylstearat, Pigmenten und Parfüm für die Herstellung von Lidschatten, Augenbrauenstiften, Puder- und Rouge-Presslingen verwendet werden. Dabei werden die wasserabweisenden Eigenschaften, sowie die verdickende Wirkung dieser Homo- und Copolymerwachse genutzt, um ein Verlaufen der Fettschminken zu unterdrücken.

Die oben beschriebenen Homo- und/oder Copolymerwachse eignen sich zur Herstellung kosmetischer, dermatologischer und pharmazeutischer Zubereitungen, besonders vorteilhaft zur Herstellung dekorativer kosmetischer Mittel, Sonnenschutzmittel, Deodorantien, Haarpflege- und Stylingmittel, Reinigungsmittel für die Haut, insbesondere Peelings, sowie Enthaarungsmittel.

Bei den erfindungsgemäßen Zubereitungen kann es sich um die unterschiedlichsten kosmetischen, pharmazeutischen und dermatologischen Formulierungen handeln. Insbesondere kann es sich um Stifte, Abdeckstifte, Akne-Stifte, Lippenstifte, Makeups, Grundierungen, Gesichtspuder, Rouge, Mascara, Lidschatten, Eye-liner, Peeling-Cremes, Haarwachse, Haar-Stylingmittel, Styling-Fluids, Haarschäume, Haargele, Haarsprays, Mousse, Haaröl, Spitzenfluids, Haarkuren, Nachtcremes, Pflegecremes, Nährcremes, Parfumcremes, Bodylotions, Salben, Lippenpflegemittel, Sonnenschutzmittel, Deodorantien, Antiperspirantien, colorierte Gele in Form von Stiften, wie z.B. Mehrphasenstiften, Sticks, Pasten, Puder, Cremes, Cremeschäume, Lotionen, selbstschäumende, schaumförmige, nachschäumende oder schäumbare Emulsionen, Gele, Roll-On-Präparate, Schäume oder Depilatorien handeln.

In einer bevorzugten Ausführungsform liegen die erfindungsgemäßen Zubereitungen in Form von Sticks oder Stiften vor.

In einer weiteren bevorzugten Ausführungsform liegen die erfindungsgemäßen Zubereitungen in Form einer Emulsion vor.

In einer besonders bevorzugten Ausführungsform werden die oben beschrieben Homo- und/oder Copolymerwachse in mikronisierter Form in Emulsionen eingearbeitet.

Bei den Emulsionen kann es sich sowohl um Wasser-in-Öl-Emulsionen als auch um Öl-in-Wasser-Emulsionen, Mikroemulsionen, Nanoemulsionen und multiple Emulsionen handeln. Die Herstellung der Emulsionen kann in bekannter Weise, d.h. beispielsweise durch Kalt-, Heiß-, Heiß/Kalt- oder PIT-Emulgierung erfolgen. Eine besonders bevorzugte Ausführungsform sind selbstschäumende, schaumförmige, nachschäumende oder schäumbare Emulsionen und Mikroemulsionen. Die erfindungsgemäßen Zubereitungen zeichnen sich durch eine besonders gute Haftung der kosmetischen Mittel auf der Haut aus und bilden hydrophobe Filme, die durch das sich bildende Hautfett kaum gelöst werden, so dass unerwünschte Farbverschiebungen der Pigmente bzw. eine Migrationen der Wirkstoffe oder festen Zusatzstoffe unterbleiben.

Mit Hilfe von Emulgatoren lassen sich eine Vielzahl von Wachzubereitungen herstellen. Die Auswahl des Emulgators ermöglicht die Herstellung von nichtionogenen und ionogenen Wachsdispersionen.

Die erfindungsgemäßen Emulsionen enthalten mindestens
a) eines der oben beschriebenen Homo- und/oder Copolymerwachse,
b) eine Ölkomponente
c) einen Emulgator
d) optional weitere Wachse.

Die Ölkomponente kann vorteilhafterweise ausgewählt werden aus den Gruppen der Mineralöle, Mineralwachse, Öle, wie Triglyceride, Fette, Wachse und andere natürliche und synthetische Fettkörper, vorzugsweise Ester von Fettsäuren mit Alkoholen niedriger C-Zahl, z.B. mit Isopropanol, Propylenglycol oder Glycerin, oder Ester von Fettalkoholen mit Alkansäuren niedriger C-Zahl mit Fettsäuren oder Alkylbenzoate.

Eine Klasse von erfindungsgemäß bevorzugten Ölen und Fetten sind die Triglyceride von linearen oder verzweigten, gesättigten oder ungesättigten, gegebenenfalls hydroxylierten C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z.B. das Handelsprodukt Myritol^{®} 318. Auch gehärtete Triglyceride sind erfindungsgemäß bevorzugt. Auch Öle tierischen Ursprungs, beispielweise Rindertalg, Perhydrosqualen oder Lanolin können eingesetzt werden.

Eine weitere Klasse von erfindungsgemäß bevorzugten Ölen und Fetten sind die Benzoesäureester von linearen oder verzweigten C₈-C₂₂-Alkanolen, z.B. die Handelsprodukte Finsolv^{®}SB (Isostearylbenzoat), Finsolv^{®}TN (C₁₂-C₁₅-Alkylbenzoat) und Finsolv^{®}EB (Ethylhexylbenzoat).

Eine weitere Klasse von erfindungsgemäß bevorzugten Ölen und Fetten sind die Dialkylether mit insgesamt 8 bis 36 Kohlenstoffatomen, insbesondere 12 bis 24 C-Atomen, wie z.B. Di-n-octylether (Cetiol^{®} OE), Di-n-decylether, Di-n-nonylether, Din-undecylether, Di-n-dodecylether, Di-3-ethyldecylether, tert-Butyl-n-octylether, isoPentyl-n-octylether, 2-Methyl-pentyl-n-octylether, n-Hexyl-n-octylether, n-Octyl-n-decylether, n-Decyl-n-undecylether, n-Undecyl-n-dodecylether und n-Hexyl-n-Undecylether sowie Di-tert-butylether oder Di-iso-pentylether.

Eine weitere Klasse von erfindungsgemäß bevorzugten Ölen und Fetten sind Kohlenwasserstofföle zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, hydrierte Polyisobutylene, Docosane, Hexadecan, Isohexadecan, Paraffinöle, Paraffinwachse, Isoparaffinöle, z.B. die Handelsprodukte der Permethyl^{®}-Serie, Squalan, Squalen, synthetische Kohlenwasserstoffe wie Polyisobuten und alicyclische Kohlenwasserstoffe, z.B. das Handelsprodukt 1,3-Di-(2-ethyl-hexyl)-cyclohexan (Cetiol^{®}S), Ozokerit, Mikrowachse und Ceresin. Ebenso in Betracht kommen verzweigte gesättigte oder ungesättigte Fettalkohole mit 6-30 Kohlenstoffatomen, z.B. Isostearylalkohol, sowie Guerbetalkohole.

Eine weitere Klasse von erfindungsgemäß bevorzugten Ölen und Fetten sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z.B. unter dem Handelsnamen Cosmacol^{®} der EniChem, Augusta Industriale.

Eine weitere Klasse von erfindungsgemäß bevorzugten Ölen und Fetten sind Dicarbonsäureester von linearen oder verzweigten C₂-C₁₀-Alkanolen, wie Di-n-butyladipat (Cetiol^{®}B), Di-(2-ethylhexyl)-adipat, Di-(2-ethylhexyl)-succinat sowie Diolester wie Ethylenglycol-dioleat, Ethylenglycol-di-isotridecanoat, Propylenglycoldi(2-ethylhexanoat), Propylenglycol-di-isostearat, Propylenglycol-di-pelargonat, Butandiol-di-isostearat und Neopentylglycoldicaprylat.

Ebenso bevorzugte Öle und Fette sind symmetrische, unsymmetrische oder cyclische Ester der Kohlensäure mit Fettalkoholen, Glycerincarbonat oder Dicaprylylcarbonat (Cetiol^{®}CC).

Eine weitere Klasse von erfindungsgemäß bevorzugten Ölen und Fetten sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

In einer weiteren bevorzugten Ausführungsform der Erfindung, wenn die Ölkomponente ein Silikonöl ist, liegen die erfindungsgemäßen Zubereitungen vorzugsweise in Form einer Wasser-in-Silikon Emulsion vor und enthalten Wasser, Silikon, einen oder mehrere Emulgatoren und ein oder mehrere Copolymerwachse.

An Silikonölen bzw. -wachsen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation) erhältlichen Dimethicone, sowie die unter SilCare® Silicone 41 M65, SilCare® Silicone 41 M70, SilCare® Silicone 41 M80 (Clariant GmbH) erhältlichen Dimethicone, Stearyldimethylpolysiloxan, C₂₀-C₂₄-Alkyl-dimethylpolysiloxan, C₂₄-C₂₈-Alkyl-dimethylpolysiloxan, aber auch die unter SilCare® Silicone 41 M40, SilCare® Silicone 41 M50 (Clariant GmbH) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Erfindungsgemäße als Emulsionen vorliegende Zubereitungen enthalten einen oder mehrere Emulgatoren aus der Gruppe der nichtionischen, anionischen, kationischen oder amphoteren Emulgatoren.

Als nichtionogene Emulgatoren stehen zur Verfügung Anlagerungsprodukte von 0 bis 30 Mol Ethylenoxid und/oder 0 bis 5 Mol Propylenoxid an lineare Fettalkohole mit 6 bis 30 C-Atomen, bevorzugt 10 bis 22 C-Atomen, und ganz besonders bevorzugt 14 bis 22 C-Atomen. Einsetzbar sind beispielsweise Octanol (Caprylalkohol), Octenol, Octadienol, Decanol (Caprinalkohol), Decenol, Decadienol, Dodecanol (Laurylalkohol), Dodecadienol, Ricinolalkohol, Myristylalkohol, Cetylalkohol, Stearylalkohol, Cetearylalkohol, Oleylalkohol, Linoleylalkohol, Linolenylalkohol, Arachidylalkohol, Behenylalkohol. Einsetzbar sind auch Fettalkoholschnitte, die durch Reduktion natürlich vorkommender Triglyceride, wie Rindertalg, Palmöl, Erdnussöl, Rüböl, Baumwollsaatöl, Sojaöl, Sonnenblumenöl und Leinöl erhalten werden oder die aus Umesterungsprodukten mit entsprechenden Alkoholen aus Fettsäureestern erzeugt werden und somit ein Gemisch von unterschiedlichen Fettalkoholen darstellen. Solche Substanzen sind beispielsweise unter der Bezeichnung Stenol^{®}, z.B. Stenol^{®} 1618 oder Lanette^{®}, z.B. Lanette^{®} O und Lanette^{®}22 oder Lorol^{®}, z.B. Lorol^{®}C18 im Handel erhältlich.

Eine weitere Klasse von erfindungsgemäß bevorzugten Emulgatoren sind Anlagerungsprodukte von 0 bis 30 Mol Ethylenoxid und/oder 0 bis 5 Mol Propylenoxid an lineare und/oder verzweigte, gesättigte und/oder ungesättigte Fettsäuren mit 6 bis 30 C-Atomen, bevorzugt 10 bis 22 Kohlenstoffatomen. Zu nennen sind Isostearinsäure, wie die Handelsprodukte Emersol^{®}871 und Emersol^{®}875, Isopalmitinsäuren wie Edenor^{®}IP95, sowie alle weiteren unter der Handelsbezeichnung Edenor^{®} (Cognis) käuflichen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeosterinsäure, Arachinsäure, Gadoleinsäure, Behensäure, Erucasäure und Dimere von ungesättigten Fettsäuren, sowie deren technische Mischungen. Besonders bevorzugt sind Fettsäureschnitte aus Cocosöl oder Palmöl. Insbesondere bevorzugt ist Stearinsäure.

Üblicherweise werden die Fettsäuren mit einem basischen Mittel, z.B. NaOH, neutralisiert und beispielsweise in Form ihrer Natrium-, Kalium-, Ammonium-, Calcium-, Magnesium- und Zink-Salze verwendet.

Eine weitere Klasse von einsetzbaren Emulgatoren sind Ester von gewünschtenfalls alkylierten Zuckern mit C₆-C₃₀-Fettsäuren. Als Zucker können beliebige Mono- oder Oligosaccharide eingesetzt werden. Üblicherweise werden Monosaccharide mit 5 oder 6 Kohlenstoffatomen eingesetzt, beispielsweise Ribose, Xylose, Lyxose, Altose, Glucose, Fructose, Galactose, Arabinose, Altrose, Mannose, Gulose, Idose, Talose, sowie die Desoxyzucker Rhamnose und Fucose. Auch Zucker mit 4 Kohlenstoffatomen können eingesetzt werden, z.B. Erythrose und Threose. Erfindungsgemäß geeignete Oligosaccharide sind aus zwei bis 10 Monosaccharideinheiten zusammengesetzt, z.B. Sucrose (Saccharose), Lactose oder Trehalose. Bevorzugte Zuckerbausteine sind die Monosaccharide Glucose, Fructose, Galactose, Arabinose und das Disaccharid Sucrose. Glucose und Sucrose sind besonders bevorzugt. Die Zucker können partiell mit Methyl, Ethyl-, Propyl-, Isopropyl- oder Butylgruppen verethert sein, z.B. Methylglucosid, Ethylglucosid oder Butylglucosid. Zur Veresterung können alle C₆-C₃₀-Fettsäuren und deren Mischungen verwendet werden, die vorstehend genannt wurden. Geeignet sind prinzipiell einfach und mehrfach veresterte Zucker. Bevorzugt sind die Mono-, Sesqui- und Diester, beispielsweise Sucrosemonostearat, Sucrosedistearat, Sucrosemonococoat, Sucrosedicocoat, Methylglucosidmonostearat, Methylglucosidsesquistearat, Methylglucosidisostearat, Ethylglucosidmonolaurat, Ethylglucosiddilaurat, Ethylglucosidmonococoat, Ethylglucosiddicocoat und Butylglucosidmonococoat.

Eine weitere Klasse geeigneter Emulgatoren sind C₈-C₂₂-Alkylmono- und -oligoglycoside entsprechend der allgemeinen Formel RO-(Z)ₓ, wobei R für eine C₈-C₂₂-Alkylgruppe, Z für Zucker und x für die Anzahl der Zuckereinheiten stehen. Die erfindungsgemäß verwendbaren Alkylmono- und -oligoglycoside können lediglich einen bestimmten Alkylrest R enthalten. Besonders bevorzugt sind solche Alkylmono- und -oligoglycoside, bei denen R im Wesentlichen aus C₈- und C₁₀₋Alkylgruppen, im Wesentlichen aus C₁₂- und C₁₄-Alkylgruppen, im Wesentlichen aus C₈- bis C₁₆-Alkylgruppen oder im Wesentlichen aus C₁₂- bis C₁₆-Alkylgruppen besteht. Als Zuckerbaustein Z können beliebige Mono- oder Oligosaccharide, wie sie vorstehend genannt wurden, eingesetzt werden. Bevorzugte Zuckerbausteine sind Glucose, Fructose, Galactose, Arabinose und Sucrose, wobei Glucose besonders bevorzugt ist. Die erfindungsgemäß verwendbaren Alkylmono- und -oligoglycoside enthalten im Schnitt 1,1 - 5, bevorzugt 1,1 - 2,0 und besonders bevorzugt 1,1 - 1,8 Zuckereinheiten.

Auch die alkoxylierten Homologen der genannten Alkylmono- und -oligoglycoside können erfindungsgemäß eingesetzt werden. Geeignet sind beispielsweise Cocoylglucosid, Decylglucosid, Laurylglucosid, Cetearylglucosid und Arachidylglucosid.

Besonders bevorzugt sind neben den genannten Alkylmono- und -oligoglucosiden auch die Gemische aus Alkylmono- und -oligoglucosiden und Fettalkoholen, z.B. die im Handel erhältlichen Produkte Montanov^{®}68 und Montanov^{®}202.

Eine weitere Klasse bevorzugter Emulgatoren sind die Partialester von Propylenglycol, Glycerin und Sorbitan mit C₈-C₂₂-Fettsäuren. Zur Veresterung können alle C₈-C₂₂-Fettsäuren und deren Mischungen verwendet werden, die vorstehend bereits genannt wurden. Besonders geeignete Beispiele sind Propylenglycolmonostearat, Glycerinmonolaurat, Glycerinmonostearat, Glycerindistearat, Glycerinmonooleat, Sorbitanmonolaurat, Sorbitandilaurat, Sorbitanmonostearat, Sorbitansesquistearat, Sorbitandistearat, Sorbitanmonoisostearat, Sorbitanmonooleat, Sorbitandioleat oder die Handelsprodukte Monomuls^{®}90-0, Monomuls^{®}90-L 12 und Cutina^{®}MD. Diese Emulgatoren können durchschnittlich bis zu 10 Ethylenoxid- und/oder Propylenoxideinheiten pro Molekül enthalten.

Eine weitere bevorzugt Klasse an Emulgatoren sind Polyglycerine der Formel HO-CH₂-CHOH-CH₂[-O-CH₂-CHOH-CH₂]ₙ-O-CH₂-CHOH-CH₂OH mit n = 0-8 und deren Ester mit linearen und verzweigten C₈-C₂₂-Fettsäuren, die in der Alkylkette funktionelle Gruppen tragen können, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI).

Eine weitere Klasse bevorzugter Emulgatoren sind Sterole (Sterine), insbesondere Cholesterol, Lanosterol, β-Sitosterol, Stigmasterol, Campesterol und Ergosterol sowie Mykosterole. Handelsübliche Sterol-Emulgatoren werden auf der Basis von Soja- oder Rapssterolen hergestellt. Erfindungsgemäß bevorzugt ist der Einsatz von Sterolen, die 5 - 10 Ethylenoxideinheiten pro Molekül enthalten. Geeignet sind z.B. die Handelsprodukte Generol^{®}122, Generol^{®} 122 E 5, Generol^{®} 122 E 10 und Generol^{®}RE 10.

Ebenso bevorzugt einsetzbare Emulgatoren sind Phospholipide, vor allem die Phosphatidylcholine oder Lecithine. Phospholipide sind Phosphorsäurediester, seltener ―monoester, von zumeist linearen gesättigten und ungesättigten C₈-C₂₂₋Fettsäuren. Bevorzugt ist Sojalecithin.

Eine weitere Klasse bevorzugter Emulgatoren sind die Veresterungsprodukte von Milchsäure oder Glykolsäure mit linearen oder verzweigten C₈-C₂₂-Fettsäuren sowie die Natrium-, Kalium-, Ammonium-, Calcium-, Magnesium- und Zink-Salze dieser Veresterungsprodukte.

Besonders bevorzugt sind Veresterungsprodukte der allgemeinen Formel (5) 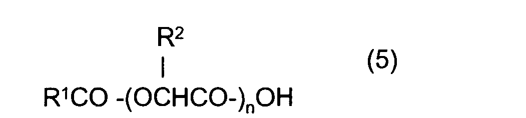 wobei R¹ einen linearen oder verzweigten gesättigten oder ungesättigten Alkylrest mit 5 bis 21 Kohlenstoffatomen und R² eine Methylgruppe oder ein Wasserstoffatom darstellen und n eine ganze Zahl von 1 - 4 ist.

Unter den Acylresten R¹CO- sind wiederum die Reste ausgewählt aus der Caproyl-, Capryloyl-, Caprinoyl-, Lauroyl-, Myristoyl-, Cetoyl-, Palmitoyl-, Stearoyl-, Isostearoyl-und der Oleyl-Gruppe bevorzugt. Besonders bevorzugt sind die Stearoyl- und die Isostearoyl-Gruppe.

Der Rest R² ist vorzugsweise Methyl.

Der Oligomerisierungsgrad n ist vorzugsweise 1 oder 2.

Insbesondere bevorzugt ist die Verbindung Natriumstearoyl-2-lactylat.

Eine weitere Klasse bevorzugt eingesetzter Emulgatoren sind Phosphorsäuremono-, -di-, und -triester von gesättigten oder ungesättigten linearen oder verzweigten Fettalkoholen mit 8 bis 30 Kohlenstoffatomen und ihre Ethylenoxidaddukte mit 1 - 10 Ethylenoxid-Gruppen pro Molekül. Diese Alkyl- und Alkenylphosphate sind in der allgemeinen Formel (6) dargestellt

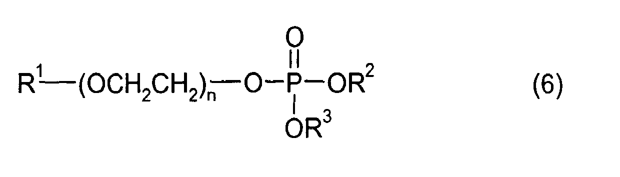

in der R¹ einen gesättigten oder ungesättigten, linearen oder verzweigten Kohlenwasserstoffrest mit 8 bis 30 Kohlenstoffatomen, R² und R³ unabhängig voneinander ein Wasserstoffatom, X oder einen Rest (CH₂CH₂O)ₙR¹, n Zahlen von 0 bis 10 und X ein Alkali- oder Erdalkalimetallkation oder ein Kation NR⁴R⁵R⁶R⁷, mit R⁴ bis R⁷ unabhängig voneinander stehend für einen C₁-C₄-Kohlenwasserstoffrest, darstellen.

Die erfindungsgemäß bevorzugten Alkyl- und Alkenylphosphate weisen als Gruppe R¹ Alkylreste mit 12 - 18 Kohlenstoffatomen auf, die gesättigt oder ungesättigt sowie linear oder verzweigt sein können. Diese Gruppen R¹ sind insbesondere Lauryl, Myristyl, Cetyl, Palmityl, Stearyl, Isostearyl und Oleyl. Bevorzugte Werte für n sind entweder 0 oder Werte von 1 - 10, bevorzugt 2 - 5, besonders bevorzugt 3 - 4 (Alkyl-oder Alkenyletherphosphate). Weiterhin bevorzugt ist die Verwendung von Estergemischen aus Mono-, Di- und Triestern, wobei der Anteil an Mono- und Diester gegenüber dem Triesteranteil überwiegt. Die Verwendung von reinen Triestern kann aber ebenfalls bevorzugt sein. Geeignete Handelsprodukte stammen z.B. aus der Hostaphat^{®}-Serie (Clariant), z.B. Hostaphat^{®}KW 340 D, Hostaphat^{®}KO300 N, Hostaphat^{®}KO380 und Hostaphat^{®}KL 340.

Eine weitere Klasse von erfindungsgemäß bevorzugt eingesetzten Emulgatoren sind Acylglutamate der Formel (7) 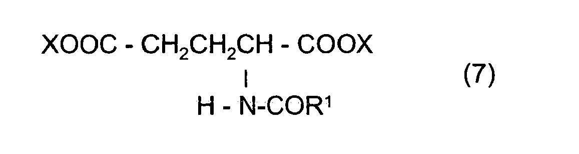 in der R¹CO für einen linearen oder verzweigten Acylrest mit 6 bis 22 Kohlenstoffatomen und 0,1,2 oder 3 Doppelbindungen und X für Wasserstoff, ein Alkali- oder Erdalkalimetallkation, für ein Ammonium, Alkylammonium, Alkanolammonium und/oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat.

Eine weitere Klasse von erfindungsgemäß bevorzugten Emulgatoren sind die Ester einer hydroxysubstituierten Di- oder Tricarbonsäure der allgemeinen Formel (8), 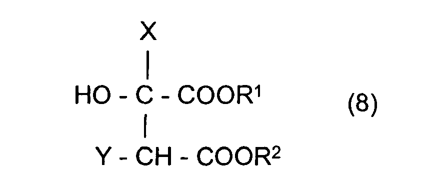 in der X H oder eine -CH₂COOR-Gruppe ist, Y H oder -OH ist unter der Bedingung, dass Y H ist, wenn X -CH₂COOR ist, R, R¹ und R² unabhängig voneinander ein Wasserstoffatom, ein Alkali- oder ein Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylsaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist.

Eine weitere Klasse von erfindungsgemäß bevorzugten Emulgatoren sind die Ester des Sulfobernsteinsäuresalzes der allgemeinen Formel (9) 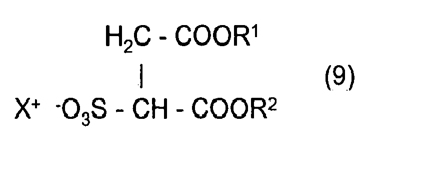 in der R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₈)-Hydroxyalkylpolyole mit 2 bis 16 Hydroxylresten ausgewählt ist, unter der Maßgabe, dass wenigstens eine der Gruppen R¹ oder R² ein Rest Z ist, und X⁺ ein Alkali- oder Erdalkalimetallkation, eine Ammoniumgruppe oder das Kation einer ammonium-organischen Base bedeutet.

Eine weitere Klasse von erfindungsgemäß bevorzugten Emulgatoren sind die Sulfobernsteinsäuremono- und -dialkylester mit 8 bis 24 C-Atomen in der Alkylgruppe und Sulfobernsteinsäuremonoalkylpolyoxyester mit 8 bis 24 C-Atomen in der Alkylgruppe und 1 bis 6 Ethoxygruppen und ihre Alkali-, Erdalkalimetall- oder Ammoniumsalze.

Eine weitere Klasse von erfindungsgemäß bevorzugten Emulgatoren sind die Ester der Weinsäure und Zitronensäure mit Alkoholen, die Anlagerungsprodukte von etwa 2 bis 10 Molekülen Ethylenoxid und/oder Propylenoxid an Fettalkohole mit 8 bis 22 C-Atomen darstellen und ihre Alkali-, Erdalkalimetall- oder Ammoniumsalze.

Weitere erfindungsgemäß bevorzugte Emulgatoren sind Ethercarbonsäuren der Formel R-O-(CH₂-CH₂O)ₓ-CH₂-COOH, in der R eine lineare Alkylgruppe mit 8 bis 30 C-Atomen und x 0 oder 1 bis 10 ist, Acylsarcosinate mit einem linearen oder verzweigten Acylrest mit 6 bis 22 Kohlenstoffatomen und 0, 1, 2 oder 3 Doppelbindungen, Acyltaurate mit einem linearen oder verzweigten Acylrest mit 6 bis 22 Kohlenstoffatomen und 0, 1, 2 oder 3 Doppelbindungen sowie Acylisethionate mit einem linearen oder verzweigten Acylrest mit 6 bis 22 Kohlenstoffatomen und 0, 1, 2 oder 3 Doppelbindungen, sowie die Alkali-, Erdalkalimetall- oder Ammoniumsalze dieser Emulgatoren.

An amphoteren Emulgatoren stehen vorzugsweise zur Verfügung Alkylaminoalkylcarbonsäuren, Betaine, Sulfobetaine und Imidazolinderivate.

Vorzugsweise sind Fettalkoholethoxylate gewählt aus der Gruppe der ethoxylierten Stearylalkohole, Cetylalkohole, Cetylstearylalkohole, insbesondere Polyethylenglykol(13)stearylether, Polyethylenglykol(14)stearylether, Polyethylenglykol(15)stearylether, Polyethylenglykol(16)stearylether, Polyethylenglykol(17)stearylether, Polyethylenglykol(18)stearylether, Polyethylenglykol(19)stearylether, Polyethylenglykol(20)stearylether, Polyethylenglykol(12)isostearylether, Polyethylenglykol(13)isostearylether, Polyethylenglykol(14)isostearylether, Polyethylenglykol(15)isostearylether, Polyethylenglykol(16)isostearylether, Polyethylenglykol(17)isostearylether, Polyethylenglykol(18)isostearylether, Polyethylenglykol(19)isostearylether, Polyethylenglykol(20)isostearylether, Polyethylenglykol(13)cetylether, Polyethylenglykol(14)cetylether, Polyethylenglykol(15)cetylether, Polyethylenglykol(16)cetylether, Polyethylenglykol(17)cetylether, Polyethylenglykol(18)cetylether, Polyethylenglykol(19)cetylether, Polyethylenglykol(20)cetylether, Polyethylenglykol(13)isocetylether, Polyethylenglykol(14)isocetylether, Polyethylenglykol(15)isocetylether, Polyethylenglykol(16)isocetylether, Polyethylenglykol(17)isocetylether, Polyethylenglykol(18)isocetylether, Polyethylenglykol(19)isocetylether, Polyethylenglykol(20)isocetylether, Polyethylenglykol(12)oleylether, Polyethylenglykol(13)oleylether, Polyethylenglykol(14)oleylether, Polyethylenglykol(15)oleylether, Polyethylenglykol(12)laurylether, Polyethylenglykol(12)isolaurylether, Polyethylenglykol(13)cetylstearylether, Polyethylenglykol(14)cetylstearylether, Polyethylenglykol(15)cetylstearylether, Polyethylenglykol(16)cetylstearylether, Polyethylenglykol(17)cetylstearylether, Polyethylenglykol(18)cetylstearylether, Polyethylenglykol(19)cetylstearylether, Polyethylenglykol(20)cetylstearylether.

Vorzugsweise sind Fettsäureethoxylate gewählt aus der Gruppe der ethoxylierten Stearate, Isostearate und Oleate, insbesondere Polyethylenglycol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(1 2)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium-laureth (11 EO)-carboxylat verwendet werden.

Als Alkylethersulfat ist Lauryldiglycolethersulfat-Natriumsalz, als ethoxyliertes Cholesterinderivat Polyethylenglykol(30)Cholesterylether vorteilhaft. Ebenso bevorzugt ist Polyethylenglykol(25)Sojasterol.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaprat/caprinat, Polyethylenglykol(20)glyceryloleat, Polyethylenglykol(20)glycerylisostearat und Polyethylenglykol(18)glyceryloleat/cocoat zu wählen.

Unter den Sorbitanestern eignen sich besonders Polyethylenglykol (20)sorbitanmonolaurat, Polyethylenglycol (20)sorbitanmonostearat, Polyethylenglycol (20)sorbitanmonoisostearat, Polyethylenglycol (20)sorbitanmonopalmitat, Polyethylenglykol (20)sorbitanmonooleat.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen, sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Insbesondere vorteilhafte W/O-Emulgatoren sind Glycerylmonostearat, Glycerylmonoisostearat, Glycerylmonomyristat, Glycerylmonooleat, Glycerylmonolaurat, Glycerylmonocaprylat, Glycerylmonocaprinat, Diglycerylmonostearat, Diglycerylmonoisostearat, Propylenglykolmonostearat, Propylenglykolmonoisostearat, Propylenglykolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol oder Polyethylenglycol(2)-stearylether.

In den erfindungsgemäßen Zubereitungen können Gemische von Verbindungen aus mehreren dieser Substanzklassen enthalten sein.

In einer weiteren besonders bevorzugten Ausführungsform liegen die erfindungsgemäßen Zubereitungen in Form von Öl-in-Wasser-Emulsionen vor.

In einer insbesondere bevorzugten Ausführungsform liegen die erfindungsgemäßen Zubereitungen als Emulsionen vom Typ Öl-in-Wasser, vorzugsweise als kosmetische oder dermatologische Emulsionen vom Typ Öl-in-Wasser, vor und enthalten bezogen auf das Gesamtgewicht der Zubereitung
a) bis zu 95 Gew.-%, vorzugsweise 60 bis 92 Gew.-%, besonders bevorzugt 70 bis 90 Gew.-%, insbesondere bevorzugt 75 bis 85 Gew.-% einer Wasserphase,
b) bis zu 40 Gew.-%, vorzugsweise 1 bis 40 Gew.-%, besonders bevorzugt 2 bis 25 Gew.-%, insbesondere bevorzugt 5 bis 20 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Homo- und/oder Copolymerwachs.

In einer weiteren insbesondere bevorzugten Ausführungsform liegen die erfindungsgemäßen Zubereitungen als Gelcremes vom Typ Öl-in-Wasser, vorzugsweise als kosmetische oder dermatologische Gelcremes vom Typ Öl-in-Wasser, vor und enthalten bezogen auf das Gesamtgewicht der Zubereitung
a) bis zu 95 Gew.-%, vorzugsweise 50 bis 95 Gew.-%, besonders bevorzugt 70 bis 90 Gew.-%, insbesondere bevorzugt 75 bis 85 Gew.-% einer Wasserphase,
b) bis zu 30 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) bis zu 5 Gew.-%, vorzugsweise 0,5 bis 5 Gew.-%, besonders bevorzugt 0,2 bis 4 Gew.-%, insbesondere bevorzugt 0,5 bis 3 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Homo- und/oder Copolymerwachs.

In einer weiteren besonders bevorzugten Ausführungsform liegen die erfindungsgemäßen Zubereitungen in Form von Wasser-in-Öl-Emulsionen vor.

In einer insbesondere bevorzugten Ausführungsform liegen die erfindungsgemäßen Zubereitungen als Emulsionen vom Typ Wasser-in-Öl, vorzugsweise als kosmetische oder dermatologische Emulsionen vom Typ Wasser-in-Öl, vor und enthalten bezogen auf das Gesamtgewicht der Zubereitungen
a) bis zu 95 Gew.-%, vorzugsweise 40 bis 95 Gew.-%, besonders bevorzugt 50 bis 90 Gew.-%, insbesondere bevorzugt 60 bis 85 Gew.-% einer Wasserphase,
b) bis zu 60 Gew.-%, vorzugsweise 2 bis 60 Gew.-%, besonders bevorzugt 5 bis 40 Gew.-%, insbesondere bevorzugt 10 bis 30 Gew.-% einer Ölphase,
c) bis zu 20 Gew.-%, vorzugsweise 0,5 bis 20 Gew.-%, besonders bevorzugt 1 bis 15 Gew.-%, insbesondere bevorzugt 4 bis 12 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Homo- und/oder Copolymerwachs.

In einer weiteren insbesondere bevorzugten Ausführungsform liegen die erfindungsgemäßen Zubereitungen als Wasser-in-Silikon-Emulsionen, vorzugsweise als kosmetische oder dermatologische Wasser-in-Silikon-Emulsionen, vor und enthalten bezogen auf das Gesamtgewicht der Zubereitung
a) bis zu 90 Gew.-%, vorzugsweise 20 bis 90 Gew.-%, besonders bevorzugt 40 bis 85 Gew.-%, insbesondere bevorzugt 60 bis 80 Gew.-% einer Wasserphase,
b) bis 80 Gew.-%, vorzugsweise 10 bis 70 Gew.-%, besonders bevorzugt 20 bis 60 Gew.-%, insbesondere bevorzugt 30 bis 50 Gew.-% an Silikonöl,
c) 0,5 bis 20 Gew.-%, vorzugsweise 1 bis 15 Gew.-%, besonders bevorzugt 3 bis 10 Gew.-% eines oder mehrerer Emulgatoren und
d) 0,01 bis 5 Gew.-%, vorzugsweise 0,05 bis 3 Gew.-%, besonders bevorzugt 0,1 bis 2 Gew.-% an Homo- und/oder Copolymerwachs.

In einer weiteren bevorzugten Ausführungsform werden die erfindungsgemäß eingesetzten Homo- und/oder Copolymerwachse in mikronisierter Form eingesetzt, vorzugsweise in Teilchengrößen von 4 bis 45 µm und insbesondere bevorzugt in Teilchengrößen von 4 bis 20 µm.

Mikronisierte Homo- und/oder Copolymerwachse können z.B. durch Feinstmahlung auf geeigneten Strahl- oder mechanischen Mühlen oder durch Versprühen aus der Schmelze erhalten werden. Die mittlere Partikelgröße (sogenannter d50-Wert) so erhaltener mikronisierter Wachse liegt in der Regel zwischen 3 und 30 µm und vorzugsweise zwischen 5 und 15 µm.

Die mikronisierten Homo und/oder Copolymerwachse lassen sich leichter dispergieren, bewirken bessere Gleiteigenschaften der Mittel und verbessern das Hautgefühl und die Verteilbarkeit der Mittel auf der Haut und auf den Haaren. Besonders vorteilhaft können die oben beschriebenen Homo und/oder Copolymerwachse in Peelings zum Reinigen und Pflegen der Haut eingearbeitet werden.

In einer weiteren bevorzugten Ausführungsform liegen die erfindungsgemäßen Zubereitungen in Form einer Dispersion vor und enthalten
a) ein Trägermaterial, vorzugsweise ein oder mehrere Ölkomponenten und/oder Lösungsmittel,
b) ein oder mehrere Emulgatoren und
c) ein oder mehrere der oben beschriebenen Homo- und/oder Copolymerwachse und gegebenenfalls ein oder mehrere weitere Wachse.

In einer weiteren bevorzugten Ausführungsform liegen die erfindungsgemäßen Zubereitungen als Wachsdispersionen vor, enthaltend
a) ein Trägermaterial, vorzugsweise ein oder mehrere Ölkomponenten und/oder Lösungsmittel,
b) ein oder mehrere Emulgatoren und
c) ein oder mehrere der oben beschriebenen Homo und/oder Copolymerwachse und
d) optional ein oder mehrere weitere Wachse
wobei der Wachsgehalt 20 bis 45 Gew.-% beträgt. Die Wachsdispersionen können in kosmetische, pharmazeutische und dermatologische Zubereitungen eingearbeitet werden.

Die erfindungsgemäßen kosmetischen, pharmazeutischen und dermatologischen Zubereitungen oder Wachsdispersionen können neben den Homo- und/oder Copolymerwachsen oder mikronisierten Homo- und/oder Copolymerwachsen weitere Wachse, wie oxidierte Polyethylenwachse, Amidwachse, Carnaubawachse, Montanwachse, Paraffinwachse, Fischer Tropsch Wachse gegebenenfalls in Kombination mit hydrophilen Wachsen, wie z.B. Cetylstearylalkohol, enthalten.

Besonders bevorzugt können die erfindungsgemäßen kosmetischen Zubereitungen als weitere Wachse Copolymerwachse enthalten, enthaltend
a) eine oder mehrere Struktureinheiten -CH₂-CHR-, wobei R für eine lineare oder verzweigte Alkylgruppe mit 24 bis 58 Kohlenstoffatomen steht,
b) gegebenenfalls eine oder mehrere Struktureinheiten 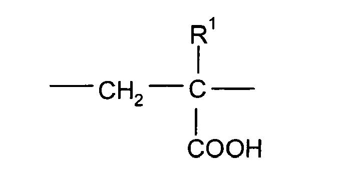 wobei R¹ für Wasserstoff oder Methyl steht,
c) eine oder mehrere Struktureinheiten 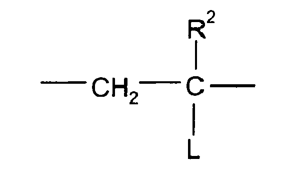 wobei
   - R²: für Wasserstoff oder Methyl steht,
   - L: -COOR³, -CONR⁷R⁸ oder -COO⁻X⁺ bedeutet,
   R³ für eine geradkettigte oder verzweigte Alkylgruppe mit 1 bis 36 Kohlenstoffatomen oder eine geradkettigte oder verzweigte Alkenylgruppe mit 2 bis 36 Kohlenstoffatomen steht, die gegebenenfalls auch alkoxyliert sein kann und vorzugsweise Ethylenoxy- (EO), Propylenoxy- (PO), Butylenoxy- (BO) oder EO/PO-Gruppen enthalten kann, oder eine Gruppe (AO)ₓ-H ist, wobei (AO) für eine Ethoxy-, Propoxy- oder Butoxygruppe steht und x eine Zahl von 1 bis 50 ist, oder für eine Glycidylgruppe, eine C₂-C₁₀-Hydroxyalkylgruppe oder für eine Glyceringruppe steht, oder
   für eine cyclische aromatische oder nichtaromatische Gruppe, vorzugsweise eine Cycloalkylgruppe, mit 5 bis 8, vorzugsweise 6, Ringatomen steht, oder für eine cyclische aromatische oder nichtaromatische Gruppe mit 5 bis 8, vorzugsweise 6, Ringatomen steht, wobei der Ring aus Kohlenstoffatomen und Heteroatomen, vorzugsweise O und/oder N, gebildet ist, und sowohl die Kohlenstoffatome, als auch die Stickstoffatome mit linearen oder verzweigten Alkyl- oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁴, worin R⁴ eine Alkylgruppe mit 1 bis 22 Kohlenstoffatomen ist, substituiert sein können, oder für ―(CR⁵R⁶)_{y}-Cycloalkyl oder für ―(CR⁵R⁶)_{y}-Aryl steht, wobei R⁵ und R⁶ jeweils unabhängig voneinander für H oder eine lineare oder verzweigte Alkylgruppe mit 1 bis 4 Kohlenstoffatomen stehen und y eine Zahl von 1 bis 10 ist, oder für eine Perfluoralkylgruppe mit 8 bis 18 Kohlenstoffatomen steht,
   R⁷ und R⁸jeweils unabhängig voneinander für Wasserstoff stehen, oder für eine geradkettigte oder verzweigte Alkylgruppe mit 1 bis 36 Kohlenstoffatomen oder eine geradkettigte oder verzweigte Alkenylgruppe mit 2 bis 36 Kohlenstoffatomen stehen, die gegebenenfalls auch alkoxyliert sein können und vorzugsweise Ethylenoxy- (EO), Propylenoxy- (PO), Butylenoxy- (BO) oder EO/PO-Gruppen enthalten können, oder
   für eine (C₂-C₁₀)-Hydroxyalkylgruppe stehen, oder
   für -CH₂-CH₂-N(CH₃)₂ oder für einen Polyaminrest stehen, oder
   für eine cyclische aromatische oder nichtaromatische Gruppe, vorzugsweise eine Cycloalkylgruppe, mit 5 bis 8, vorzugsweise 6, Ringatomen stehen, oder
   für eine cyclische aromatische oder nichtaromatische Gruppe mit 5 bis 8, vorzugsweise 6, Ringatomen stehen, wobei die Ringe aus Kohlenstoffatomen und Heteroatomen, vorzugsweise O und/oder N, gebildet sind, und sowohl die Kohlenstoffatome, als auch die Stickstoffatome mit linearen oder verzweigten Alkyl-oder Alkoxygruppen mit 1 bis 36 Kohlenstoffatomen oder mit linearen oder verzweigten Alkenyl- oder Alkenyloxygruppen mit 2 bis 36 Kohlenstoffatomen, oder mit Acetylgruppen -COR⁹, worin R⁹ eine Alkylgruppe mit 1 bis 22 Kohlenstoffatomen ist, substituiert sein können, oder
   R⁷ und R⁸ zusammen mit dem Stickstoffatom, an das sie gebunden sind, einen 5-, 6-oder 7-gliedrigen aromatischen oder nichtaromatischen Ring bilden, und die Ringe neben dem Stickstoffatom vorzugsweise lediglich CH₂-Gruppen enthalten,
   X⁺ für Li⁺, Na⁺, K⁺, Mg⁺⁺/2, Ca⁺⁺/2, Al⁺⁺⁺/3, NH₄⁺, ein Monoalkylammonium-, Dialkylammonium-, Trialkylammonium- und/oder Tetraalkylammoniumion steht,
   wobei es sich bei den Alkylsubstituenten der Ammoniumionen unabhängig voneinander um (C₁-C₂₂)-Alkylreste oder (C₂-C₁₀)-Hydroxyalkylreste handeln kann, und
d) gegebenenfalls eine oder mehrere Struktureinheiten, die sich von Styrol, 3-Methylstyrol, 4-Methylstyrol oder α-Methylstyrol ableiten.

In einer weiteren bevorzugten Ausführungsform liegen die erfindungsgemäßen Zubereitungen als dekorative Mittel vor.

Eine weitere bevorzugte Ausführungsform sind kosmetische und dermatologische Stifte, beispielsweise Lippenstifte, Sonnencremestifte, Antiacne-Stifte, Augenbrauenstifte, Abdeckstifte und Deo-Stifte, enthaltend
a) eine Lipidphase aus mindestens einer Ölkomponente und mindestens einem Homo- und/oder Copolymerwachs, wie oben beschrieben,
b) optional in der Lipidphase lösliche oder dispergierbare Substanzen,
c) eine wässrige Phase,
d) optional in Wasser lösliche oder dispergierbare Substanzen,
e) optional eine oder mehrere Wirksubstanzen, und
f) mindestens einen W/O-Emulgator,
wobei der Anteil der wässrigen Phase, bezogen auf die fertige Zubereitung, 30 bis 80 Gew.-% betragen kann.

In einer weiteren bevorzugten Ausführungsform, insbesondere wenn es sich um dekorative Mittel handelt, enthalten die erfindungsgemäßen kosmetischen und dermatologischen Zubereitungen ein oder mehrere Farbmittel, vorzugsweise ausgewählt aus Farblacken, Toner und Pigmenten. Hierbei liegen sie vorzugsweise in Form von Pudern, Preßlingen, Pasten, Cremes oder Sticks vor.

In einer besonders bevorzugten Ausführungsform liegen die erfindungsgemäßen Zubereitungen in Form von Suspensionen vor und enthalten bezogen auf das Gesamtgewicht der Zubereitungen
a) 0,1 bis 10 Gew.-%, vorzugsweise 0,2 bis 6 Gew.-%, besonders bevorzugt 0,3 bis 5 Gew.-% an Homo- und/oder Copolymerwachs und
b) 0,1 - 30 Gew.-%, vorzugsweise 0,5 bis 15 Gew.-%, besonders bevorzugt 1,0 bis 10 Gew.-% an festen Partikeln, insbesondere ausgewählt aus der Gruppe der Farbstoffe, farbgebenden Pigmente, Effekt- und Lichtschutzpigmente, Adsorbentien und Abrassivkomponenten.

In einer weiteren besonders bevorzugten Ausführungsform liegen die erfindungsgemäßen Zubereitungen als Lidschatten auf Gelbasis vor und enthalten bezogen auf das Gesamtgewicht der Zubereitungen
a) 0,1 bis 10 Gew.-%, vorzugsweise 0,2 bis 6 Gew.-%, besonders bevorzugt 0,3 bis 5 Gew.-% an Homo- und/oder Copolymerwachs und
b) 0,1 bis 30 Gew.-%, vorzugsweise 0,5 bis 15 Gew.-%, besonders bevorzugt 1,0 bis 10 Gew.-% an Farbstoffen und/oder farbgebenden Pigmenten.

Die erfindungsgemäßen Zubereitungen können feste anorganische und organische Partikel enthalten. Für dekorative Kosmetika werden farbige und auch farblose Pigmente eingesetzt. Einige der nachfolgend genannten Pigmente dienen auch als UV-Absorber bzw. Lichtschutzpigmente.

Die Farbstoffe- und -pigmente, sowohl organische als auch anorganische Farbstoffe, können aus der entsprechenden Positivliste der Kosmetikverordnung bzw. der EG-Liste kosmetischer Färbemittel ausgewählt werden. Folgende Substanzen können beispielsweise in den erfindungsgemäßen Zubereitungen eingesetzt werden.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1'-naphthylazo]-1-hydroxy-7-aminoaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethyl-N-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3,-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha, beta- bzw, gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Manganammoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium- und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z.B. Paprikaextrakte, β-Carotin und Cochenille.

Vorteilhaft eingesetzt werden auch Perlglanzpigmente, z.B. Fischsilber (Guanin/ Hypoxanthin-Mischkristalle aus Fischschuppen) und Perlmutt (vermahlene Muschelschalen), monokristalline Perlglanzpigmente wie z.B. Bismuthoxychlorid (BiOCl), Schicht-Substrat Pigmente, z.B. Glimmer/Metalloxid, silberweiße Perlglanzpigmente aus TiO₂, Interferenpigmente (TiO₂, unterschiedliche Schichtdicke), Farbglanzpigmente (Fe₂O₃) und Kombinationspigmente (TiO₂/Fe₂O₃, TiO₂/Cr₂O₃, TiO₂/Berliner Blau, TiO₂/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt.

Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z.B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z.B. Bismutoxychlorid (BiOCl), sowie die Oxide von beispielsweise Titan, insbesondere die TiO₂-Modifikationen Anatas und Rutil, Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z.B. von 2 - 50 µm, 5 - 25 µm, 5 - 40 µm, 5 - 60 µm, 5 - 95 µm, 5-100 µm, 10 - 60 µm, 10 - 100 µm, 10 - 125 µm, 20 - 100 µm, 20 - 150 µm, sowie < 15 µm. Eine breitere Teilchengrößenverteilung z.B. von 20 - 150 µm, ruft glitzernde Effekte hervor, während eine engere Teilchengrößenverteilung von < 15 µm für eine gleichmäßige seidige Erscheinung sorgt.

Die erfindungsgemäßen Zubereitungen enthalten Effektpigmente vorzugsweise in Mengen von 0,1 - 20 Gew.-%, besonders bevorzugt 0,5 - 10 Gew.-% und insbesondere bevorzugt 1 - 5 Gew.-%, jeweils bezogen auf das Gesamtgewicht der Zubereitung.

Bei den bevorzugten anorganischen Lichtschutzpigmenten handelt es sich um feindisperse oder kolloiddisperse Metalloxide und Metallsalze, beispielsweise Titanoxid, Zinkoxid, Eisenoxid, Aluminiumoxid, Ceroxid, Zirkoniumoxid, Silikate (Talk) und Bariumsulfat. Die Partikel sollten dabei einen mittleren Durchmesser von weniger als 100 nm, vorzugsweise zwischen 5 und 50 nm und besonders bevorzugt zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®}T2000 (Merck). Als hydrophobe Coatingmittel kommen dabei vor allem Silicone und dabei speziell Trialkoxyocytylsilane oder Simethicone in Frage. Besonders bevorzugt sind Titandioxid und Zinkoxid.

Die bevorzugten anorganischen Partikelsubstanzen sind hydrophil oder amphiphil. Vorteilhafterweise können sie oberflächlich beschichtet, insbesondere oberflächlich wasserabweisend behandelt sein. Beispiele hiefür sind mit Aluminiumstearat beschichtete Titanoxid-Pigmente, mit Dimethylpolysiloxan (Dimethicone) beschichtetes Zinkoxid, mit Dimethicone beschichtetes Bornitrid und mit einem Gemisch aus Dimethylpolysiloxan und Silicagel und Aluminiumoxidhydrat beschichtetes Titanoxid, mit Octylsilanol beschichtetes Titanoxid oder sphärische Polyalkylsesquioxan-Partikel.

Bei organischen Lichtschutzpigmenten handelt es sich um bei Raumtemperatur kristallin vorliegende Substanzen, die in der Lage sind, ultraviolette Strahlen zu absorbieren und die aufgenommene Energie in Form längerwelligerer Strahlung, z.B. Wärme, wieder abzugeben. Man unterscheidet UVA-Filter und UVB-Filter. Die UVA-und UVB-Filter können sowohl einzeln als auch in Mischungen eingesetzt werden. Die erfindungsgemäß geeigneten organischen UV-Filter sind ausgewählt aus den bei Raumtemperatur festen Derivaten von Dibenzoylmethan, Zimtsäureestern, Diphenylsäureestern, Benzophenon, Campher, p-Aminobenzoesäureestern, o-Aminobenzoesäureestern, Salicylsäureestern, Benzimidazolen, 1,3,5-Triazinen, monomeren und oligomeren 4,4-Diarylbutadiencarbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein. Erfindungsgemäß besonders bevorzugte öllösliche UV-Filter sind (1-(4-tert-Butylphenyl)-3-(4'-methoxyphenyl)propan-1,3-dion, 1-Pheny)-3-(4'-Isopropylphenyl)-propan-1,3-dion, 3-(4'-Methylbenzyliden)-D,L-campher, 4-(Dimethylamino)-benzoesäure-2-ethylhexylester, 4-(Dimethylamino)-benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-octylhexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino(p-carbo-2'-ethyl-1'-hexyloxy)-1,3,5-triazin (Octyl Triazone) und Dioctyl Butamido Triazone sowie beliebige Mischungen der genannten Komponenten.

Bevorzugte wasserlösliche UV-Filter sind 2-Phenylbenzimidazol-5-sulfonsäure und deren Alkali, Erdalkali, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z.B. 4-(2-oxo-3-bornylidenmethyl)benzolsulfonsäure und 2-Methyl-5-(2-oxo-3-bornyliden)sulfonsäure und deren Salze.

In den erfindungsgemäßen Zubereitungen sind die anorganischen und organischen Lichtschutzpigmente in Mengen von vorzugsweise 0,1 bis 30 Gew.-%, besonders bevorzugt 1 bis 20 Gew.-% und insbesondere bevorzugt 2 bis 15 Gew.-%, jeweils bezogen auf das Gesamtgewicht der Zubereitung, enthalten.

Die erfindungsgemäßen Zubereitungen können teilchenförmige anorganische oder organische Adsorbentien mit mittleren Partikeldurchmessern von 1 - 100 µm, enthalten. Die Adsorbentien sind ausgewählt aus pyrogenen Kieselsäuren, z.B. den Aerosol-Typen, Fällungskieselsäuren, Kieselgelen, Siliciumdioxid, Tonen, z.B. Bentonite oder Kaolin, Magnesiumaluminiumsilikaten, z.B. Talkum und Bornitrid, gegebenenfalls modifizierte Stärken und Stärkederivate, Cellulosepulvern, Lactoglobulinderivaten, Polymerpulvern aus Polyolefinen, Polycarbonaten, Polyurethanen, Polyamiden, Polyestern, Polystyrolen, Polyacrylaten, (Meth)acrylat-oder (Meth)acrylat-Vinyliden-Copolymeren, die vernetzt sein können, Teflon oder Siliconen, sowie Mischungen der genannten Substanzen.

Die erfindungsgemäßen Zubereitungen können Abrasivkomponenten enthalten, beispielsweise gemahlene Pflanzenteile wie Mandelkleie oder Weizenkleie, kristalline Cellulose, gehärtetes Jojobaöl, Polymerkügelchen, bevorzugt aus Polyethylen oder Polyamid-11, mit mittleren Durchmessern von 90-600 µm, und aus wirkstoffhaltigen Mikro- oder Millikapseln, die petrochemische Polymere (z.B. aus Polyamid wie Nylon-11) und/oder Biopolymere wie Gelatine, Pektin, Pflanzlichen Gummen, Alginaten und Carrageenan enthalten. Bevorzugt eingesetzt werden Mandelkleie, Weizenkleie, gehärtetes Jojobaöl und Polyethylenkügelchen.

In einer bevorzugten Ausführungsform der Erfindung handelt es sich um make-up, Augen-make-up, Mascara, eyeliner und Rouge, gekennzeichnet durch besondere Wasserfestigkeit, Farbbrillianz, Perlglanzeffekt, gute Hautsensorik und gute Verteilbarkeit der Mittel auf der Haut.

In einer weiteren bevorzugten Ausführungsform der Erfindung handelt es sich um Nagellack mit ausgezeichneten Glanzeffekten.

In einer weiteren bevorzugten Ausführungsform enthalten die erfindungsgemäßen Zubereitungen eine oder mehrere UV-Lichtschutzfilter. Bei diesen erfindungsgemäßen Zubereitungen handelt es sich vorzugsweise um Sonnenschutzmittel. Die Sonnenschutzmittel liegen vorzugsweise in Form von Sprays, Sticks, Pasten, Gelen oder Lotionen vor.

Als UV-Filter kommen vorzugsweise in Betracht 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon;
2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäureisoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-aminocarbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat, N,N,N-Trimethyl-4-(2-oxoborn-3-ylidenemethyl) anilium methyl sulphate, Homosalate (INN) Oxybenzone (INN) 2-Phenylbenzimidazole-5-sulfonsäure und ihre Na, K, und Triethanolaminsalze, alpha-(2-Oxoborn-3-ylidene) toluol-4-sulfonsäure und ihre Salze, Octyl methoxyzimtsäure, Isopentyl-4-methoxyzimtsäure, Isoamyl p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometriazole Trisiloxane) benzoesäure, 4,4-((6-(((1,1-dimethylethyl)amino)carbonyl)phenyl)amino)-1,3,5-triazine-2,4-diyl)diimino)bis-,bis(2-ethylhexyl)ester) benzoesäure, 4,4-((6-(((1,1-dimethylethyl)amino)-carbonyl)phenyl)amino)-1,3,5-triazine-2,4-diyl)diimino)bis-, bis(2-ethylhexyl)ester) 3-(4'-Methylbenzylidene)-d-1 camphor (4-Methylbenzylidene Camphor) 3-Benzylidene camphor (3-Benzylidene camphor) 2-Ethylhexyl salicylat (Octyl Salicylat) 4-Dimethyl-aminobenzoat von ethyl-2-hexyl (octyl dimethyl PABA), 2-Hydroxy-4-methoxybenzo-phenone-5-sulfonisäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat Di-p-methoxy zimtsäure, p-Amino-benzoesäure und Ester, 4-tert-Butyl-4'-methoxydibenzoylmethan, 4-(2-β-glucopyranoxy)propoxy-2-hydroxybenzophenon, Octyl Salicylat, Methyl-2,5-düsopropyl zimtsäure, Cinoxat, Dihydroxydimethoxybenzophenon, Dinatriumsalz von 2,2'-dihydroxy-4,4'-dimethoxy-5,5'-disulfobenzophenon, Dihydroxy benzophenon, 1-3,4-Dimethoxyphenyl)-4,4-dimethyl-1,3-pentanedion, 2-Ethylhexyl dimethoxybenzylidene dioxoimidazolidin propionat, Tetrahydroxybenzophenone, Terephthalylden dicamphor sulfonsäure, 2,4,6-tris[4-2-ethylhexyloxycarbonyl)anilino]-1,3,5-triazin, Methyl bis(trimethylsiloxy)silyl isopentyl trimethoxy-zimtsäure, Amyl p-dimethylamino benzoat, 2-Ethylhexyl p-dimethylamino benzoat, Isopropyl p-methoxyzimtsäure/ Diisopropylzimtsäureester, 2-Ethylhexyl p-methoxyzimtsäure, 2-Hydroxy-4-methoxy benzophenon, 2-Hydroxy-4-methoxybenzophenon-5-sulfsäure und das Trihydrat, 2-Hydroxy-4-methoxybenzophenon-5-sulfonat, Na-Salz und Phenyl-benzimidazol-sulfonsäure.

Weitere geeignete UV-Filter sind ausgewählt aus den bei Raumtemperatur festen Derivaten von Dibenzoylmethan, Zimtsäureestern, Diphenylsäureestern, Benzophenon, Capher, p-Aminobenzoesäureestern, o-Aminobenzoesäureestern, Salicylsäureestern, Benzimidazolen, 1,3,5-Triazinen, monomeren und oligomeren 4,4-Diarylbutadien-carbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein. Erfindungsgemäß besonders bevorzugte öllösliche UV-Filter sind 1-Phenyl-3-(4'-Isopropylphenyl)-propan-1,3-dion, (Dimethylamino)benzoesäure-2-octylester, 4-(Dimethylamino)-benzoesäureamylester, 4-Methoxyzimtsäure-2-ethylhexylester, 4-Methoxyzimtsäure-propylester, 4-Methoxyzimtsäureisopentylester, 2-Cyano-3,3-phenylzimtsäure-2-ethylhexylester (Octocrylene), Salicylsäure-4-isopropylbenzylester, Salicylsäurehomomenthylester (3,3,5-Trimethyl-octylhexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone sowie beliebige Mischungen der genannten Komponenten. Bevorzugte wasserlösliche UV-Filter sind 2-Phenylbenzimidazol-5-sulfonsäure und deren Alkali, Erdalkali, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z.B. 4-(2-oxo-3-bornylidenmethyl)-benzolsulfonsäure und 2-Methyl-5-(2-oxo-3-bornyliden)sulfonsäure und deren Salze.

Die erfindungsgemäßen Zubereitungen, beispielsweise die Sonnenschutzmittel, enthalten UV-Lichtschutzfilter in den Gewichtsmengen von vorzugsweise 0,1 bis 10 %, besonders bevorzugt von 0,5 bis 8 % und insbesondere bevorzugt von 1 bis 5 %, bezogen auf die fertigen Zubereitungen.

Die Sonnenschutzmittel können als UV-Lichtschutzfilter aber z.B. auch die unter der oben aufgeführten Gruppe der Pigmente genannten anorganischen UV-Absorber und Lichtschutzpigmente enthalten. Dies sind in den erfindungsgemäßen Zubereitungen vorzugsweise in Mengen von 0,1 bis 30 Gew.-%, besonders bevorzugt in Mengen von 0,5 bis 15 Gew.-% und insbesondere bevorzugt in Mengen von 1,0 bis 10 Gew.-%, jeweils bezogen auf das Gesamtgewicht der Zubereitungen, enthalten.

In einer weiteren bevorzugten Ausführungsform enthalten die erfindungsgemäßen Zubereitungen, beispielsweise die Sonnenschutzmittel, ein oder mehrere Antioxidantien.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. (α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den erfindungsgemäßen Zubereitungen beträgt vorzugsweise 0,001 bis 30 Gew.-%, besonders bevorzugt 0,05 bis 20 Gew.-% und insbesondere bevorzugt 1 bis 10 Gew.-%, bezogen auf das Gesamtgewicht der Zubereitungen.

Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der Zubereitung, zu wählen. Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Zubereitung, zu wählen.

In einer besonders bevorzugten Ausführungsform der Erfindung enthalten die kosmetischen oder pharmazeutischen Zubereitungen Antioxidantien ausgewählt aus Superoxid-Dismutase, Tocopherol (Vitamin E) und Ascorbinsäure (Vitamin C).

In einer weiteren bevorzugten Ausführungsform handelt es sich bei den erfindungsgemäßen Zubereitungen um Deodorantien und Antiperspirantien, die ein oder mehrere Substanzen ausgewählt aus antimikrobiell wirkenden Substanzen, Adstringentien und deodorierenden Stoffen enthalten. Diese Zubereitungen liegen vorzugsweise in Form von Sprays, Sticks, Pasten, Gelen oder Lotionen vor.

Bevorzugt geeignet als antimikrobielle Wirkstoffe sind Cetyltrimethylammoniumchlorid, Cetylpyridiniumchlorid, Benzethoniumchlorid, Düsobutylethoxyethyl-dimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol und Kombinationen dieser Wirksubstanzen.

Die erfindungsgemäßen Zubereitungen enthalten die antimikrobiellen Wirkstoffe bevorzugt in Mengen bis 50 Gew.-%, besonders bevorzugt in Mengen von 0,01 bis 10 Gew.-% und insbesondere bevorzugt in Mengen von 0,1 bis 10 Gew.-%, bezogen auf die fertigen Zubereitungen.

Bevorzugte Adstringentien sind Oxide, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink.

Die erfindungsgemäßen Zubereitungen enthalten die adstringenden Wirkstoffe bevorzugt in Mengen von 0 bis 50 Gew.-%, besonders bevorzugt in Mengen von 0,01 bis 10 Gew.-% und insbesondere bevorzugt in Mengen von 0,1 bis 10 Gew.-%, bezogen auf die fertigen Zubereitungen.

Als deodorierende Stoffe bevorzugt sind Allantoin und Bisabolol. Diese werden vorzugsweise in Mengen von 0,0001 bis 10 Gew.-% eingesetzt.

In einer weiteren bevorzugten Ausführungsform handelt es sich bei den erfindungsgemäßen Zubereitungen um Peelings. Diese liegen vorzugsweise in Form von Peeling-Cremes oder -gelen zum Reinigen und Glätten der Haut vor.

In einer weiteren bevorzugten Ausführungsform handelt es sich bei den erfindungsgemäßen Zubereitungen um Zahnpflegemittel, insbesondere um Zahnpflegemittel, die die oben genannten Homo- und Copolymerwachse in mikronisierter Form als Abrassivkomponente enthalten. Die Zahnpflegemittel können als Zahnpasta, Zahncreme oder als Zahngel vorliegen

In einer weiteren bevorzugten Ausführungsform handelt es sich bei den erfindungsgemäßen Zubereitungen um Enthaarungsmittel.

Die erfindungsgemäßen Zubereitungen können als weitere Hilfs- und Zusatzstoffe andere pulverförmige Stoffe, Füllkörper, kationische Polymere, Filmbildner, Verdickungs- und Dispergiermittel, Überfettungsmitfel, feuchtigkeitsspendende Mittel, Stabilisatoren, biogene Wirkstoffe, Glycerin, Konservierungsmittel, Perlglanzmittel, Duftstoffe, Lösungsmittel, Trübungsmittel, weitere Wachse, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, Erfrischungsmittel, beispielsweise Methylacetat, Stoffe mit keratolytischer und keratoplastischer Wirkung, Enzyme und Trägersubstanzen enthalten.

Des weiteren können als Füllkörper SiO₂, Silica, ZnO, Kaolin, mit SiO₂ modifiziertes Kaolin, Polytetrafluorethylen, Nylon, Talkum, Glimmer, Polymethylmethacrylat, Polyethylen, Polyether, Polycarbonate, Polyvinylchlorid, Polystyrol, Polyamide, Polyurethane, Polyacrylate, natürliche Polymere, Seidenpulver, mikrokristalline Cellulose, natürliche organische Verbindungen wie verkapselte oder unverkapselte Getreidestärke und Gemische davon eingesetzt werden.

An kationischen Polymeren stehen die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guar-hydroxypropyltriammoniumchloride, sowie Calciumalginat und Ammoniumalginat zur Verfügung. Ebenso geeignet sind kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan.

Geeignete Silikonverbindungen sind beispielsweise Dimethylpolysiloxan, Methylphenylpolysiloxane, cyclische Silikone und amino-, fettsäure-, alkohol-, polyether-, epoxy-, fluor- und/oder alkylmodifizierte Silikonverbindungen, sowie Polyalkylsiloxane, Polyalkylarylsiloxane, Polyethersiloxan-Copolymere, wie in US 5,104,645 und den darin zitierten Schriften beschrieben, die bei Raumtemperatur sowohl flüssig als auch harzförmig vorliegen können.

Geeignete Filmbildner sind, je nach Anwendungszweck Salze der Phenylbenzimidazolsulfonsäure, wasserlösliche Polyurethane, beispielsweise C₁₀-Polycarbamyl-Polyglycerolester, aber auch Polyvinylalkohol, Polyvinylpyrrolidon, sowie Copolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carbocyvinyl-Polymere, wie Carbomere und deren Salze, Polysaccharide, beispielsweise Polydextrose und Glucan.

Als Überfettungsmittel können Substanzen wie beispielsweise Lanilin, Lecithin, polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Als feuchtigkeitsspendende Substanz stehen beispielsweise Isopropylpalmitat, Glycerin und/oder Sorbitol zu Verfügung, die bevorzugt in den Mengen von 0,1 bis 50 Gew.-% eingesetzt werden.

Als biogene Wirkstoffe können beispielsweise Pflanzenextrakte, beispielsweise Aloe Vera und Vitaminkomplexe, Bisabolol^{®}, Allantoin^{®}, Phytantriol^{®}, Panthenol^{®}, AHA-Säuren, Lokalanästhetika, Antibiotika, Antiphlogistika, Antiallergica, Corticosteoide, Sebostatika, Phanthenol, Allantoin und Proteine eingesetzt werden.

Als Konservierungsmittel eignen sich beispielsweise Phenoxyethanol, Diazolidinylharnstoff, Parabene, Pentandiol, butyliertes Hydroxytoluol, butyliertes Hydroxyanisol oder Sorbinsäure. Sie werden vorzugsweise in den Mengen von 0,001 bis 5 Gew.-%, besonders bevorzugt von 0,01 bis 3 Gew.-% und insbesondere bevorzugt von 0,1 bis 2 Gew.-%, bezogen auf die fertigen Zubereitungen, eingesetzt.

Als Farbstoffe können die für kosmetische und pharmazeutische Zwecke geeigneten und zugelassenen Substanzen verwendet werden.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z.B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykoldistearate mit durchschnittlich 3 Glykoleinheiten.

Sofern die erfindungsgemäßen Zubereitungen perlglanzgebende Verbindungen enthalten, sind diese bevorzugt in einer Menge von 0,1 bis 15 Gew.-% und besonders bevorzugt in einer Menge von 1 bis 10 Gew.-% in den erfindungsgemäßen Zubereitungen enthalten.

Als antifungizide Wirkstoffe eignen sich bevorzugt Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Zn-Pyrethion und Octopyrox.

Als Verdickungs- und Dispergiermittel besonders geeignet sind Ethylenglykolester von Fettsäuren mit 14 bis 22, besonders bevorzugt 16 bis 22, Kohlenstoffatomen, insbesondere Mono- und Di-ethylenglykolstearat. Ebenfalls bevorzugt geeignet sind Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl-(C₁₂-C₂₂)-amidobenzoesäure und deren lösliche Salze, N,N-Dihydrocarbyl-(C₁₆-C₁₈)-amidobenzoesäure und deren lösliche Salze und N,N-di(C₁₆-C₁₈)-amidobenzoesäure und deren Derivate. Weiterhin besonders geeignet sind Polyacrylate und Carbomere, insbesondere solche wasserlöslicher oder wasserquellbarer Copolymerisate auf Basis von Acrylamidoalkylsulfonsäuren und N-Vinylcarbonsäureamiden.

Zur Erhöhung der Farbintensität können die erfindungsgemäßen Zubereitungen die in kosmetischen Systemen üblichen Carrier enthalten, insbesondere Benzylalkohol, Vanillin (4-Hydroxy-3-methoxy-benzaldehyd), Isovanillin, p-Hydroxyanisol, 3-Hydroxy-4-methoxy-benzaldehyd, 2-Phenoxyethanol, Salicylaldehyd, 3,5-Dihydroxybenzaldehyd, 3,4-Dihydroxybenzaldehyd, 4-Hydroxyphenylacetamid, p-Hydroxybenzoesäure-methylester, p-Hydroxybenzaldehyd, m-Kresol, Hydrochinonmonomethylether, o-Fluorphenol, m-Fluorphenol, p-Fluorphenol, 2-(2'-Hydroxyphenoxy)-ethanol, 3,4-Methylendioxyphenol, Resorcinmonomethylether, 3,4-Dimethoxyphenol, 3-Trifluormethylphenol, Resorcinmonoacetat, Ethylvanillin, 2-Thiophenethanol, Milchsäurebutylester und Glykolsäurebutylester. Besonders vorteilhaft mit synergistischer Wirkung sind erfindungsgemäße Zubereitungen enthaltend Phenoxyethanol oder/oder Benzylalkohol.

Als Solubilisatoren eignen sich prinzipiell alle ein- oder mehrwertigen Alkohole und ethoxylierten Alkohole. Bevorzugt werden Alkohole mit 1 bis 4 Kohlenstoffatomen, wie z.B. Ethanol, Propanol, Isopropanol, n-Butanol und Iso-Butanol, Glycerin und deren Mischungen eingesetzt. Weiterhin bevorzugt sind Polyethylenglykole mit einer relativen Molekülmasse unter 2000. Insbesondere bevorzugt sind Polyethylenglykole mit einer relativen Molekülmasse zwischen 200 und 600 in Mengen bis zu 45 Gew.-% und Polyethylenglykole mit einer relativen Molekülmasse zwischen 400 und 600 in Mengen von 0,5 bis 15 Gew.-%. Weitere geeignete Lösungsmittel sind beispielsweise Triacetin (Glycerintriacetat) und 1-Methoxy-2-propanol.

Als Stabilisatoren können Metallsalze von Fettsäuren, wie z.B. Magnesium-, Aluminium- und/oder Zinkstearat eingesetzt werden.

Die erfindungsgemäßen Zubereitungen können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Hydroxycitronellal, Lilial und Bourgeonal, zu den Ketonen z.B. die lonone, alpha-Isomethylionon und Methylcedrylketon, zu den Alkoholen Anethol, Citronellol, Eugenol, Geranion, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Weitere Zusatzstoffe können Silikonverbindungen sein, vorzugsweise Dimethylpolysiloxan, Methyl-phenylpolysiloxane, cyclische Silikone sowie amino-, fettsäure-, alkohol-, polyether-, epoyx-, fluor- und/oder alkylmodifizierte Silikonverbindungen, beispielsweise Alkylsilicone SilCare® Silicone 41 M10, SilCare® Silicone 41 M15, SilCare® Silicone 41 M20, SilCare® Silicone 41 M30 (Clariant), Alkyltrimethicone SilCare® 31 M30, SilCare® 31 M40, SilCare® 31 M 50, SilCare® 31 M 60 (Clariant), Phenyltrimethicone SilCare® 15M30, SilCare® 15M40, SilCare® 15M50, SilCare® 15M60 (Clariant), Polyalkylarylsiloxane und Polyethersiloxan-Copolymere.

Die erfindungsgemäßen Zubereitungen können die oben genannten Silikonverbindungen vorzugsweise in den Gewichtsmengen von 0,1 bis 20 Gew.-%, besonders bevorzugt von 0,2 bis 15 Gew.-% und insbesondere bevorzugt von 0,5 bis 10 Gew.-% bezogen auf die fertigen Zubereitungen enthalten.

Die Zubereitungen besitzen üblicherweise einen pH-Wert im Bereich von 2 bis 12 und bevorzugt in einem Bereich von 3 bis 8.

Die nachfolgenden Beispiele und Anwendungen sollen die Erfindung näher erläutern, ohne sie jedoch darauf zu beschränken (bei allen Prozentangaben handelt es sich um Gewichtsprozente).

Die in Tabelle 1 aufgeführten Metallocen-Polyolefinwachse wurden nach dem in EP-A-0 571 882 angegebenen Verfahren aus den Monomeren Ethylen und/oder Propylen hergestellt. Der Gewichtsanteil der Monomeren ist der Tabelle 1 zu entnehmen.

**Tabelle 1: Zusammensetzung der Polyolefinwachse**

| Polyolefinwachs | Ethylen [Gew.-%] | Propylen [Gew.-%] |
|---|---|---|
| 1 | 100 | 0 |
| 2 | 95 | 5 |
| 3 | 0 | 100 |
| 4 | 10 | 90 |

Es wurde der Tropfpunkt, die Viskosität, das gewichtsmittlere Molekulargewicht Mw und die Dichte der Polyolefinwachse 1 bis 4 bestimmt. Die Ergebnisse sind in Tabelle 2 wiedergegeben.

**Tabelle 2: Erweichungs-/Tropfpunkt, Viskosität, gewichtsmittlere Molekulargewichte und Dichte der Polyolefinwachse**

| Polyolefinwachs | Produkttyp | Tropfpunkt [°C] | Viskosität bei 170°C [mPa.s] | gewichtsmittleres Molekulargewicht Mw [g/mol] | Dichte [g/cm³] |
|---|---|---|---|---|---|
| 1 | Ethylen-Homopolymerwachs (Metallocen) | 128 | 100 | 4200 | 0,98 |
| 2 | Ethylen-Propylen-Copolymerwachs (Metallocene) | 109 | 500 | 6800 | 0,93 |
| 3 | Propylen-Homopolymerwachs (Metallocen) | 150 | 120 | 4500 | 0,89 |
| 4 | Propylen-Ethylen-Copolymerwachs (Metallocen) | 90 | 150 | 5800 | 0,88 |

Es wurde die Pastenhärte einer Zubereitung enthaltend Polyolefinwachs 2 und die Pastenhärte zweier entsprechender Zubereitungen, die aber anstelle von Polyolefinwachs 2 handelsübliche Wachse enthalten, bestimmt und miteinander verglichen. Die Ergebnisse sind in Tabelle 3 wiedergegeben.

**Tabelle 3: Pastenhärte eines Polyolefinwachses hergestellt mittels Metallocenkatalyse im Vergleich zu Polyolefinwachsen, hergestellt mit einem Ziegler-Natta Katalysator (Licowax PE 520) oder hergestellt durch radikalische Polymerisation unter Hochdruckbedingungen (A-C 8)**

| | | | |
|---|---|---|---|
| Paraffinwachs | 17,1 | 17,1 | 17,1 |
| Ozokerit 2089 | 0,9 | 0,9 | 0,9 |
| Polyolefinwachs 2 | 7 | - | - |
| Licowax PE 520 | - | 7 | - |
| A-C 8 | - | - | 7 |
| white spirit | ad 100 | ad 100 | ad 100 |
| Pastenhärte [g/cm²] 25°C | 2460 | 420 | 1880 |
| Pastenhärte [g/cm²] 40°C | 530 | <50 | 475 |
| Pastenhärte [g/cm²] 50°C | 310 | <50 | 120 |
| Gießtemperatur [°C] | 66 | 70 | 70 |

Die Ergebnisse der Tabelle 3 zeigen, dass Polyolefinwachse, hergestellt unter Metallocenkatalyse zur Herstellung von Sticks und Stiften, beispielsweise Lippenstiften, sehr gut geeignet sind.

Formulierungsbeispiele

### Beispiel 1: W/O-Creme

| | | |
|---|---|---|
| A | Hostacerin^{®} DGI | 6,0 % |
| | Magnesiumstearat | 1,0 % |
| | Mineralöl, niedrigviskos | 5,0 % |
| | Vaseline | 10,0 % |
| | Cetiol^{®} V | 5,0 % |
| | | |
| B | 1,2-Propylenglycol | 3,0 % |
| | Wasser dest. | ad 100 % |
| | Konservierungsmittel | q.s. |
| | | |
| C | Duftstoff | 0,4 % |
| | | |
| D | Polyolefinwachs1, mikronisiert | 3,0% |

### Herstellung:

- I: Schmelzen von A bei 80°C
- II: Erwärmen von B auf 80°C
- III: Einrühren von II in I
- IV: Rühren bis Temperatur von 35°C erreicht ist
- V: Zugabe von C zu IV bei 35°C
- VI: Einrühren von D in V bei Raumtemperatur

### Beispiel 2: O/W-Creme

| | | | |
|---|---|---|---|
| A | Hostacerin^{®} DGI | | 2,0 % |
| | SilCare® Silicone 31 M30 | Clariant | 4,0 % |
| | Perliquidum | | 4,0 % |
| | Polyolefinwachs 2 | | 2,0 % |
| | Eutanol G | Clariant | 4,0 % |
| | Isopropylpalmitat | Clariant | 4,0 % |
| | Carbopol 980 | | 0,7 % |
| | | | |
| B | Hostapon^{®} KCG | | 0,6 % |
| | Natronlauge (10% in Wasser) | | 2,1 % |
| | Konservierungsmittel | | q.s. |
| | Duftstoff | | 0,4 % |
| | Wasser dest. | | ad 100 % |

### Herstellung:

- I: Erwärmen von A auf 80°C
- II: Erwärmen von B auf 80°C
- III: Emulgieren durch langsames Einrühren von B in A.

### Beispiel 3: Antiperspirant

| | | | |
|---|---|---|---|
| A | Locron^{®} L | Clariant | 10,0 % |
| | Ethanol | | 50,0 % |
| | Farnesol | | 0,5 % |
| | Duftstoff | | 0,2 % |
| | Polyolefinwachs 1 | | 0,5 % |
| | Wasser dest. | | ad 100 % |
| | Extrapon Avocado special | | 1,5 % |

### Herstellung:

Mischen der Komponenten A

### Beispiel 4: W/O-Antiperspirant Creme

| | | |
|---|---|---|
| A | Abil EM90 | 2,0 % |
| | Abil B8839 | 20,0 % |
| | Polyolefinwachs 1 | 2,0 % |
| | | |
| B | Aloxicoll L | 17,0 |
| | Wasser dest. | ad 100 % |
| | Parfümöl | q.s. |
| | Konservierungsmittel | q.s. |

### Herstellung:

- I: Phase B langsam unter Rühren bei Raumtemperatur zur Phase A geben.
- II: Homogenisieren

### Beispiel 5: Deo-Stick

| | | | |
|---|---|---|---|
| A | OCTOPIROX^{®} | (Clariant) | 0,1 % |
| | Polyolefinwachs 2 | | 3,0 % |
| | Propylene Glycol | | 71,0 % |
| | Rewoderm 66E | | 5,0 % |
| | Natriumstearat | | 5,0 % |
| | GENAPOL^{®} HS 020 | (Clariant) | 1,0 % |
| | Wasser dest. | | ad 100 % |

### Herstellung:

- I: Mischen der Komponenten A bei 50°C und rühren bis die Lösung klar ist
- II: Abfüllen und Abkühlen

### Beispiel 6: Alkoholfreier Deodorant-Roll-on (opak)

| | | |
|---|---|---|
| A | Tegodeo CW 90 | 2,0 % |
| | Polyethylenglycol(3)laurylether | 1,0 % |
| | Triethanolamin | 1,0 % |
| | | |
| B | Polyolefinwachs 2 | 1,2 % |
| | Wasser dest. | ad 100 % |
| | | |
| C | Tagat R 40 | 3,0 % |
| | Parfümöl | q.s. |
| | Konservierungsmittel | q.s. |
| | | |
| D | Zitronensäure (50 %ig in Wasser) | 0,2 % |

### Herstellung:

- I: Phasen A und B separat auf 80°C erwärmen
- II: Phase B in Phase A einrühren und homogenisieren
- III: Unter langsamem Rühren abkühlen
- IV: Bei 30°C Phase C hinzufügen
- V: Den pH-Wert mit Hilfe von Phase D einstellen

### Beispiel 7: Grundierung

| | | | |
|---|---|---|---|
| A | Nexbase® 2004 FG | | 9,0 % |
| | Myritol^{®} 318 | | 5,0 % |
| | Almond Oil | | 4,0 % |
| | SilCare^{®} Silicone 31 M40 | (Clariant) | 4,0 % |
| | SilCare^{®} Silicone 41 M15 | (Clariant) | 3,0 % |
| | Genapol^{®} HS020 | (Clariant) | 1,6 % |
| | Genapol^{®} HS200 | (Clariant) | 2,4 % |
| | Polyolefinwachs 1 | | 2,0 % |
| | | | |
| B | Vanclay^{®} | | 1,5% |
| | Talc | | 3,0 % |
| | Iron Oxide Pigments | | 7,9 % |
| | | | |
| C | Glycerin | | 5,0 % |
| | Wasser dest. | | ad 100 % |
| | Aristoflex^{®} AVC | (Clariant) | 0,4% |
| | | | |
| D | Duftstoff | | q.s. |
| | Nipaguard^{®} PDU | (Clariant) | q.s. |

### Herstellung:

- I: Mischen und schmelzen der Komponenten A bei 70°C
- II: Zugabe von B zu I bei 70°C unter Rühren
- III: Mischen von C bis Aristoflex^{®} AVC gelöst ist und Erwärmen auf 70°C
- IV: Zugabe von C zu II unter Rühren und Homogenisieren.
- V: Zugabe von D zu IV bei < 40°C

### Beispiel 8: Mascara

| | | | |
|---|---|---|---|
| A | Tylose^{®} H 4000 G4 | | 0,7 % |
| | 1,2-Propylenglykol | | 1,0 % |
| | Wasser dest. | | ad 100 % |
| | | | |
| B | Triethanolamine 99 % | | 1,2 % |
| | | | |
| C | Stearinsäure | | 3,0 % |
| | SilCare^{®} Silicone 41 M15 | (Clariant) | 1,0 % |
| | SilCare^{®} Silicone 31 M40 | (Clariant) | 2,0 % |
| | Tegocare^{®}450 | | 4,0 % |
| | Nexbase^{®} 2006 | | 2,0 % |
| | Bienenwachse | | 2,5 % |
| | Candelilla Wachs | | 2,5 % |
| | Polyolefinwachs 1 | | 3,5 % |
| | | | |
| D | Pigmente | | 10,0 % |
| | | | |
| E | Nipagin^{®} M | (Clariant) | 0,2 % |
| | Nipasol^{®} M | (Clariant) | 0,1 % |
| | | | |
| F | Duftstoff | | q.s |

### Herstellung:

- I: Komponenten A bei Raumtemperatur unter Rühren aufquellen; auf 85°C e rwärmen
- II: Zugabe von B zu A und rühren
- III: Schmelzen der Komponenten C auf 85°C
- IV: Zugabe von D zu III unter Rühren bei 85°C
- V: Zugabe von II zu IV unter kräftigem Rühren (15 Minuten bei 85°C, weitere 15 Minuten ohne Erwärmen)
- VI: Zugabe von E und F zu V bei 35 bis 40°C
- VII: Abfüllen bei 35°C bis 40°C.

### Beispiel 9: Zahncreme

| | | |
|---|---|---|
| A | Wasser dest. | ad 100 % |
| | Glycerin | 20,0 % |
| | Natriumbenzoat | 2,5 % |
| | Saccharin (10%ig) | 1,0 % |
| | Aromaöl | 1,0 % |
| | Cosmenyl Grün GG | 0,005 % |
| | Medialan^{®} LD | 6,6 % |
| | | |
| B | Tylose H 20 | 2,4 % |
| | | |
| C | Aerosil^{®} 300 | 2,0 % |
| | Polyolefinwachs 1 mikronisiert | 32,0 % |

### Herstellung:

- I: Mischen und Rühren der Komponenten A
- II: Einrühren von B in I
- III: Nacheinander Einrühren der Komponenten C zu II

### Beispiel 10: Zahncreme

| | | |
|---|---|---|
| A | Wasser dest. | ad 100 % |
| | Glycerin | 20,0 % |
| | Natriumbenzoat | 2,5 % |
| | Saccharin (10%ig) | 1,0 % |
| | Aromaöl | 1,0 % |
| | Cosmenyl Grün GG | 0,005 % |
| | Medialan^{®} LD | 6,6 % |
| | | |
| B | Tylose H 20 | 2,4 % |
| | | |
| C | Aerosil^{®} 300 | 2,0 % |
| | Polyolefinwachs 3, mikronisiert | 32,0 % |

### Herstellung:

- I: Mischen und Rühren der Komponenten A
- II: Einrühren von B in I
- III: Nacheinander Einrühren der Komponenten C zu II

### Beispiel 11: Enthaarungsmittel

| | | |
|---|---|---|
| A | Paraffinöl | 12,0 % |
| | Synthetischer Gummi SIS&SBS | 28,0 % |
| | Piccotac 1095 | 50,0 % |
| B | Polyolefinwachs 4 | 10,0 % |

### Herstellung:

- I: Aufschmelzen der Kompoenten A bei 200°C und Homogenisieren
- II: Zugabe von B zu I bei 200°C und Homogenisieren. Auftragen auf Streifenfolien.

### INCI Bezeichnung der eingesetzten Handelsprodukte:

| | | |
|---|---|---|
| Abil B8839 | | Cyclopentasiloxan/Cyclohexasiloxan |
| Abil EM90 | | Cetyldimethicon/Copolyol |
| A-C 8 | (Honeywell) | Polyethylenwachs |
| Aerosil^{®} 200 | (Degussa) | Kieselgel |
| Aerosil^{®} 300 | (Degussa) | Kieselgel |
| Aloxicoll L. | | Alumiunium Chlorohydrat |
| Aristoflex^{®} AVC | (Clariant) | Ammonium Acyloyldimethyltaurate/VP |
| | | Copolymer |
| Carbopol 980 | | Polyacrylat |
| Cetiol^{®} V | (Cognis) | Decyloleat |
| Eutanol G | | 2-Octyldodecanol |
| Extrapon^{®} | (Dragoco) | Pflanzliche Extrakte |
| Extrapon Avocado special | | Wasser/ |
| | | Ethoxydiglycol/Propylenglycol/ |
| | | Butylenglycol/Persea Gratissima |
| | | Extrakt |
| GENAPOL^{®} HS 020 | (Clariant) | Steareth-2 |
| Genapol^{®} HS200 | (Clariant) | Steareth-20 |
| Hostacerin^{®} DGI | | Polyglyceryl-2-Sesquiisostearat |
| Hostapon^{®} KCG | | Natriumcocoylglutamat |
| Licowax^{®} PE 520 | (Clariant) | Polypropylenwachs aus |
| | | Ziegler-Synthese, |
| Locron^{®} L | (Clariant) | Aluminium Chlorohydrat |
| Medialan^{®} LD | (Clariant) | Natriumlauroylsarcosinat |
| Myritol^{®} 318 | | Capric/Caprylic Triglyceride |
| Nexbase^{®} 2004 FG | | Hydrogenated Poly-1-Decene |
| Nexbase^{®} 2006 | | Poly-1-Decen |
| Nipagin^{®}M | (Clariant) | Methylparaben |
| Nipaguard^{®} PDU | (Clariant) | Propylene Glycol (und) Diazolidinyl |
| | | Urea (und) Methylparaben (und) |
| | | Propylparaben |
| Nipasol^{®}M | (Clariant) | Propylparaben |
| OCTOPIROX^{®} | (Clariant) | Piroctone Olamine |
| Piccotac 1095 | (Eastman) | Piperylene/Butene/Pentene |
| | | Copolymer |
| Rewoderm 66E | | Isostearate |
| SilCare® Silicone 31 M30 | (Clariant) | Caprylyl Trimethicone |
| SilCare® Silicone 31 M40 | (Clariant) | Caprylyl Trimethicone |
| SilCare® Silicone 41 M15 | (Clariant) | Caprylylmethicon |
| SIS&SBS | | Styrol-Isopren-Styrol&Styrol-Butadien- |
| | | Styrol |
| Tagat R40 | | PEG-40 hydrogeniertes Castoröl |
| Tegocare^{®} 450 | | Polyglyceryl-3 Methylglucose |
| | | Distearat |
| Tegodeo CW 90 | | Zinkricinoleat/Tetrahydroxypropyl- |
| | | Ethylendiamin/Laureth- |
| | | 3/Propylenglycol |
| Tylose ^{®}CB | | Carboxymethylcellulose |
| Tylose^{®} H 20 | | Hydroxyethylcellulose |
| Tylose^{®} H 4000 G4^{®} | | Hydroxyethylcellulose |
| Tylose^{®} H 100000 YP2 | | Hydroxyethylcellulose |
| Vanclay^{®} | | Kaolin |
| White spirit | | aliphatischer Kohlenwasserstoff |

Es wurden folgende Meßmethoden verwendet:
- Tropfpunkt:: DIN 51801/2
- Erweichungspunkt:: Ring/Kugel nach DIN EN 1427
- Schmelzviskosität:: DIN 53019 mit einem Rotationsviskosimeter
- Glasübergangstemperatur:: nach DIN 51700 mit Hilfe der Differentialthermoanalyse
- Kristallisationsgrad:: Der Kristallisationsgrad wird über die Schmelzwärme berechnet. Diese wird mit Hilfe der Differential Scanning Calorimetry (DSC) bestimmt. Als Referenzwert für 100% kristallines Polyethylen wird 292 J/g, für ideal kristallines Polypropylen 207 J/g verwendet.
- Isotaktischer Index:: Bestimmung durch IR-Spektroskopie nach J.P. Luongo, J. Appl. Polm. Chem., 3, 302 (1960)

Das Molmassengewichtsmittel Mw, das zahlenmittlere Molekulargewicht Mn und der resultierende Quotient Mw/Mn wurden durch Gelpermeationschromatographie bei 135°C in 1,2-Dichlorbenzol ermittelt.

Pastenhärte: Die Pastenhärte gibt die Masse in Gramm an, mit der ein Stempel mit der Fläche von 1 cm² belastet werden muß, um in die Paste einzudringen. Beschrieben ist diese Methode in Seifen-Öle-Fette-Wachse, 83, S. 595 (1957).
- Dichte (bei 23°C in g/cm³):: DIN 53479
- Nadelpenetrationszahl (NPZ) (in 0,1 mm):: DIN 51 579; DGF-M-III 9B (98); ASTM D 1321

## Patentansprüche

1. Kosmetische, pharmazeutische oder dermatologische Zubereitung enthaltend ein oder mehrere Homo- und/oder Copolymerwachse aus den Monomeren Ethylen und/oder Propylen, **dadurch gekennzeichnet, dass** die Homo- und Copolymerwachse ein gewichtsmittleres Molekulargewicht Mw von kleiner oder gleich 25000 g/mol, ein zahlenmittleres Molekulargewicht Mn von kleiner oder gleich 15000 g/mol, eine Molmassenverteilung Mw/Mn im Bereich von 1,5 bis 10, vorzugsweise von 1,5 bis 5, besonders bevorzugt von 1,5 bis 3 und inbesondere bevorzugt von 2 bis 2,5 besitzen und durch Metallocenkatalyse erhalten worden sind und wobei die Copolymerwachse, bezogen auf das Gesamtgewicht der Copolymerwachse, 0,1 bis 30,0 Gew.-% an Struktureinheiten hervorgegangen aus dem einen Monomer und 70,0 bis 99,9 Gew.-% an Struktureinheiten hervorgegangen aus dem anderen Monomer enthalten.

2. Zubereitung nach Anspruch 1 enthaltend ein oder mehrere Homopolymerwachse aus dem Monomer Ethylen, **dadurch gekennzeichnet, dass** die Homopolymerwachse ein gewichftsmittleres Molekulargewicht Mw von kleiner oder gleich 25000 g/mol, vorzugsweise von 1000 bis 22000 g/mol und besonders bevorzugt von 4000 bis 20000 g/mol, ein zahlenmittleres Molekulargewicht Mn von kleiner oder gleich 15000 g/mol, vorzugsweise von 500 bis 12000 g/mol und besonders bevorzugt von 1000 bis 5000 g/mol und eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5 besitzen.

3. Zubereitung nach Anspruch 1 enthaltend ein oder mehrere Copolymerwachse aus den Monomeren Ethylen und Propylen, **dadurch gekennzeichnet, dass** die Copolymerwachse ein gewichtsmittleres Molekulargewicht Mw von kleiner oder gleich 25000 g/mol, vorzugsweise von 2000 bis 22000 g/mol und besonders bevorzugt von 4000 bis 20000 g/mol, ein zahlenmittleres Molekulargewicht Mn von kleiner oder gleich 15000 g/mol, vorzugsweise von 1000 bis 12000 g/mol und besonders bevorzugt von 2000 bis 10000 g/mol und eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5 besitzen und wobei die Copolymerwachse, bezogen auf das Gesamtgewicht der Copolymerwachse, 70,0 bis 99,9 Gew.-% an Struktureinheiten hervorgegangen aus dem Monomer Ethylen und 0,1 bis 30,0 Gew.-% an Struktureinheiten hervorgegangen aus dem Monomer Propylen enthalten.

4. Zubereitung nach Anspruch 1 enthaltend ein oder mehrere Homopolymerwachse aus dem Monomer Propylen, **dadurch gekennzeichnet, dass** die Homopolymerwachse ein gewichtsmittleres Molekulargewicht Mw von kleiner oder gleich 25000 g/mol, vorzugsweise von 2000 bis 22000 g/mol und besonders bevorzugt von 4000 bis 22000 g/mol, ein zahlenmittleres Molekulargewicht Mn von kleiner oder gleich 15000 g/mol, vorzugsweise von 1000 bis 12000 g/mol und besonders bevorzugt von 2000 bis 10000 g/mol, eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5 und einen isotaktischen Index von größer als 70 %, vorzugsweise von 75 bis 95 % und besonders bevorzugt von 80 bis 90 % besitzen.

5. Zubereitung nach Anspruch 1 enthaltend ein oder mehrere Copolymerwachse aus den Monomeren Ethylen und Propylen, **dadurch gekennzeichnet dass** die Copolymerwachse ein gewichtsmittleres Molekulargewicht Mw von kleiner oder gleich 25000 g/mol, vorzugsweise von 2000 bis 22000 g/mol und besonders bevorzugt von 4000 bis 20000 g/mol, ein zahlenmittleres Molekulargewicht Mn von kleiner oder gleich 15000 g/mol, vorzugsweise von 1000 bis 12000 g/mol und besonders bevorzugt von 2000 bis 10000 g/mol, eine Molmassenverteilung Mw/Mn im Bereich von 1,5 bis 10, vorzugsweise von 1,5 bis 5, besonders bevorzugt von 1,5 bis 3 und insbesondere bevorzugt von 2 bis 2,5, besitzen und wobei die Copolymerwachse, bezogen auf das Gesamtgewicht der Copolymerwachse, 0,1 bis 30,0 Gew.-% an Struktureinheiten hervorgegangen aus dem Monomer Ethylen und 70,0 bis 99,9 Gew.-% an Struktureinheiten hervorgegangen aus dem Monomer Propylen enthalten.

6. Zubereitung nach einem oder mehreren der Ansprüche 1 bis 5, **dadurch gekennzeichnet, dass** die Homo- und/oder Copolymerwachse aus den Monomeren Ethylen und/oder Propylen einen Tropf- oder Erweichungspunkt Ring/Kugel zwischen 80 und 165°C haben und eine Schmelzviskosität, gemessen bei einer Temperatur von 170°C, zwischen 20 und 40000 mPa.s besitzen.

7. Zubereitung nach einem oder mehreren der Ansprüche 1 bis 6, **dadurch gekennzeichnet, dass** die Copolymerwachse aus den Monomeren Ethylen und Propylen eine Glasübergangstemperatur von maximal -20°C aufweise.

8. Zubereitung nach einem oder mehreren der Ansprüche 1 bis 7, **dadurch gekennzeichnet, dass** die Homo- und/oder Copolymerwachse aus den Monomeren Ethylen und/oder Propylen einen Kristallisationsgrad von mehr als 55 % und vorzugsweise zwischen 60 und 90 % aufweisen.

9. Zubereitung nach einem oder mehreren der Ansprüche 1 bis 8, **dadurch gekennzeichnet, dass** die Homo- und/oder Copolymerwachse aus den Monomeren Ethylen und/oder Propylen einen Kristallisationsgrad unterhalb von 55 % und vorzugsweise unterhalb von 50 % aufweise.

10. Zubereitung nach einem oder mehreren der Ansprüche 1 bis 9, **dadurch gekennzeichnet, dass** sie ein oder mehrere Homo- und/oder Copolymerwachse, die nicht polar modifiziert sind, und zusätzlich ein oder mehrere Homo- und/oder Copolymerwachse, die polar modifiziert sind, enthält.

11. Zubereitung nach einem oder mehreren der Ansprüche 1 bis 9, **dadurch gekennzeichnet, dass** die Homo- und/oder Copolymerwachse aus nicht polar modifizierten Homo- und/oder Copolymerwachsen ausgewählt sind.

12. Zubereitung nach einem oder mehreren der Ansprüche 1 bis 9, **dadurch gekennzeichnet, dass** die Homo- und/oder Copolymerwachse aus polar modifizierten Homo- und/oder Copolymerwachsen ausgewählt sind.

13. Zubereitung nach einem oder mehreren der Ansprüche 1 bis 12, **dadurch gekennzeichnet, dass** es sich um einen Stift, Abdeckstift, Akne-Stift, Lippenstift, ein Make-up, eine Grundierung, ein Gesichtspuder, Rouge, eine Mascara, einen Lidschatten, Eye-liner, eine Peeling-Creme, ein Haarwachs, Haar-Stylingmittel, Styling-Fluid, einen Haarschaum, ein Haargel, Haarspray, eine Mousse, ein Haaröl, ein Spitzenfluid, eine Haarkur, Nachtcreme, Pflegecreme, Nährcreme, Parfumcreme, Bodylotion, Salbe, ein Lippenpflegemittel, Sonnenschutzmittel, Deodorant, Antiperspirant, coloriertes Gel in Form eines Stifts wie z.B. eines Mehrphasenstifts, einen Stick, eine Paste, ein Puder, eine Creme, einen Cremeschaum, eine Lotion, eine selbstschäumende, schaumförmige, nachschäumende oder schäumbare Emulsion, ein Gel, Roll-On-Präparat, einen Schaum oder ein Depilatorium handelt.

14. Zubereitung nach Anspruch 13, **dadurch gekennzeichnet, dass** sie in Form eines Sticks oder Stifts vorliegt.

15. Zubereitung nach einem oder mehreren der Ansprüche 1 bis 14, **dadurch gekennzeichnet, dass** sie in Form einer Emulsion vorliegt.

16. Zubereitung nach Anspruch 15, **dadurch gekennzeichnet, dass** sie in Form einer Öl-in-Wasser-Emulsion vorliegt.

17. Zubereitung nach Anspruch 16, **dadurch gekennzeichnet, dass** sie als Emulsion vom Typ Öl-in-Wasser, vorzugsweise als kosmetische oder dermatologische Emulsion vom Typ Öl-in-Wasser, vorliegt und bezogen auf das Gesamtgewicht der Zubereitung
a) bis zu 95 Gew.-%, vorzugsweise 60 bis 92 Gew.-%, besonders bevorzugt 70 bis 90 Gew.-%, insbesondere bevorzugt 75 bis 85 Gew.-% einer Wasserphase,
b) bis zu 40 Gew.-%, vorzugsweise 1 bis 40 Gew.-%, besonders bevorzugt 2 bis 25 Gew.-%, insbesondere bevorzugt 5 bis 20 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Homo- und/oder Copolymerwachs
enthält.

18. Zubereitung nach Anspruch 16, **dadurch gekennzeichnet, dass** sie als Gelcreme vom Typ Öl-in-Wasser, vorzugsweise als kosmetische oder dermatologische Gelcreme vom Typ Öl-in-Wasser, vorliegt und bezogen auf das Gesamtgewicht der Zubereitung
a) bis zu 95 Gew.-%, vorzugsweise 50 bis 95 Gew.-%, besonders bevorzugt 70 bis 90 Gew.-%, insbesondere bevorzugt 75 bis 85 Gew.-% einer Wasserphase,
b) bis zu 30 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) bis zu 5 Gew.-%, vorzugsweise 0,5 bis 5 Gew.-%, besonders bevorzugt 0,2 bis 4 Gew.-%, insbesondere bevorzugt 0,5 bis 3 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Homo- und/oder Copolymerwachs
enthält.

19. Zubereitung nach Anspruch 15, **dadurch gekennzeichnet, dass** sie in Form einer Wasser-in-Öl-Emulsion vorliegt.

20. Zubereitung nach Anspruch 19, **dadurch gekennzeichnet, dass** sie als Emulsion vom Typ Wasser-in-Öl, vorzugsweise als kosmetische oder dermatologische Emulsion vom Typ Wasser-in-Öl, vorliegt und bezogen auf das Gesamtgewicht der Zubereitung
a) bis zu 95 Gew.-%, vorzugsweise 40 bis 95 Gew.-%, besonders bevorzugt 50 bis 90 Gew.-%, insbesondere bevorzugt 60 bis 85 Gew.-% einer Wasserphase,
b) bis zu 60 Gew.-%, vorzugsweise 2 bis 60 Gew.-%, besonders bevorzugt 5 bis 40 Gew.-%, insbesondere bevorzugt 10 bis 30 Gew.-% einer Ölphase,
c) bis zu 20 Gew.-%, vorzugsweise 0,5 bis 20 Gew.-%, besonders bevorzugt 1 bis 15 Gew.-%, insbesondere bevorzugt 4 bis 12 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Homo- und/oder Copolymerwachs
enthält.

21. Zubereitung nach einem oder mehreren der Ansprüche 1 bis 20, **dadurch gekennzeichnet, dass** die Homo- und/oder Copolymerwachse in mikronisierter Form eingesetzt werden.

22. Zubereitung nach einem oder mehreren der Ansprüche 1 bis 21, **dadurch gekennzeichnet, dass** sie in Form einer Dispersion vorliegt und
a) ein Trägermaterial, vorzugsweise ein oder mehrere Ölkomponenten und/oder Lösungsmittel,
b) einen oder mehrere Emulgatoren und
c) neben den in Anspruch 1 genannten Homo- und/oder Copolymerwachsen gegebenenfalls ein oder mehrere weitere Wachse
enthält.

23. Zubereitung nach einem oder mehreren der Ansprüche 1 bis 22, **dadurch gekennzeichnet, dass** sie als dekoratives Mittel vorliegt.

24. Zubereitung nach einem oder mehreren der Ansprüche 1 bis 23, vorzugsweise in Form eines Puders, Preßlings, einer Paste, Creme oder eines Sticks, **dadurch gekennzeichnet, dass** sie ein oder mehrere Farbmittel, vorzugsweise ausgewählt aus Farblacken, Toner und Pigmenten, enthält.

25. Zubereitung nach einem oder mehreren der Ansprüche 1 bis 24, **dadurch gekennzeichnet, dass** sie einen oder mehrere UV-Lichtschutzfilter enthält, vorzugsweise ein Sonnenschutzmittel ist, und besonders bevorzugt in Form eines Sprays, Sticks, einer Paste, eines Gels oder einer Lotion vorliegt.

26. Zubereitung nach einem oder mehreren der Ansprüche 1 bis 25, **dadurch gekennzeichnet, dass** sie ein oder mehrere Antioxidantien enthält.

27. Zubereitung nach einem oder mehreren der Ansprüche 1 bis 22 und 24 bis 26, vorzugsweise in der Form eines Sprays, Sticks, einer Paste, eines Gels oder einer Lotion, **dadurch gekennzeichnet, dass** sie ein Deodorant oder Antiperspirant ist und ein oder mehrere Substanzen ausgewählt aus antimikrobiell wirkenden Substanzen, Adstringentien und deodorierenden Stoffen enthält.

28. Zubereitung nach Anspruch 27, **dadurch gekennzeichnet, dass** sie ein oder mehrere deodorierende Stoffe enthält und diese ausgewählt sind aus Allantoin und Bisabolol.

29. Zubereitung nach einem oder mehreren der Ansprüche 1 bis 22 und 24 bis 26, **dadurch gekennzeichnet, dass** sie ein Peeling ist.

30. Zubereitung nach einem oder mehreren der Ansprüche 1 bis 26, **dadurch gekennzeichnet, dass** sie ein Zahnpflegemittel ist.

31. Zubereitung nach einem oder mehreren der Ansprüche 1 bis 22 und 24 bis 26, **dadurch gekennzeichnet, dass** sie ein Enthaarungsmittel ist.

## Claims

1. A cosmetic, pharmaceutical or dermatological preparation comprising one or more homopolymer and/or copolymer waxes of the monomers ethylene and/or propylene, wherein the homopolymer and copolymer waxes have a weight-average molecular weight Mw of less than or equal to 25 000 g/mol, a number-average molecular weight Mn of less than or equal to 15 000 g/mol, a molar mass distribution Mw/Mn in the range from 1.5 to 10, preferably from 1.5 to 5, particularly preferably from 1.5 to 3 and especially preferably from 2 to 2.5, and have been obtained by metallocene catalysis and where the copolymer waxes, based on the total weight of the copolymer waxes, comprise 0.1 to 30.0% by weight of structural units originating from the one monomer and 70.0 to 99.9% by weight of structural units originating from the other monomer.

2. A preparation as claimed in claim 1, comprising one or more homopolymer waxes of the monomer ethylene, wherein the homopolymer waxes have a weight-average molecular weight Mw of less than or equal to 25 000 g/mol, preferably of 1000 to 22 000 g/mol and particularly preferably of 4000 to 20 000 g/mol, a number-average molecular weight Mn of less than or equal to 15 000 g/mol, preferably of 500 to 12 000 g/mol and particularly preferably of 1000 to 5000 g/mol and a molar mass distribution Mw/Mn in the range from 1.5 to 10, preferably from 1.5 to 5, particularly preferably from 1.5 to 3 and especially preferably from 2 to 2.5.

3. A preparation as claimed in claim 1, comprising one or more copolymer waxes of the monomers ethylene and propylene, wherein the copolymer waxes have a weight-average molecular weight Mw of less than or equal to 25 000 g/mol, preferably of 2000 to 22 000 g/mol and particularly preferably of 4000 to 20 000 g/mol, a number-average molecular weight Mn of less than or equal to 15 000 g/mol, preferably of 1000 to 12 000 g/mol and particularly preferably of 2000 to 10 000 g/mol and a molar mass distribution Mw/Mn in the range from 1.5 to 10, preferably from 1.5 to 5, particularly preferably from 1.5 to 3 and especially preferably from 2 to 2.5 and where the copolymer waxes, based on the total weight of the copolymer waxes, comprise 70.0 to 99.9% by weight of structural units originating from the monomer ethylene and 0.1 to 30.0% by weight of structural units originating from the monomer propylene.

4. A preparation as claimed in claim 1, comprising one or more homopolymer waxes of the monomer propylene, wherein the homopolymer waxes have a weight-average molecular weight Mw of less than or equal to 25 000 g/mol, preferably of 2000 to 22 000 g/mol and particularly preferably of 4000 to 22 000 g/mol, a number-average molecular weight Mn of less than or equal to 15 000 g/mol, preferably of 1000 to 12 000 g/mol and particularly preferably of 2000 to 10 000 g/mol, a molar mass distribution Mw/Mn in the range from 1.5 to 10, preferably from 1.5 to 5, particularly preferably from 1.5 to 3 and especially preferably from 2 to 2.5 and have an isotactic index of greater than 70%, preferably of 75 to 95% and particularly preferably of 80 to 90%.

5. A preparation as claimed in claim 1, comprising one or more copolymer waxes of the monomers ethylene and propylene, wherein the copolymer waxes have a weight-average molecular weight Mw of less than or equal to 25 000 g/mol, preferably of 2000 to 22 000 g/mol and particularly preferably of 4000 to 20 000 g/mol, a number-average molecular weight Mn of less than or equal to 15 000 g/mol, preferably of 1000 to 12 000 g/mol and particularly preferably of 2000 to 10 000 g/mol, a molar mass distribution Mw/Mn in the range from 1.5 to 10, preferably from 1.5 to 5, particularly preferably from 1.5 to 3 and especially preferably from 2 to 2.5, and where the copolymer waxes, based on the total weight of the copolymer waxes, comprise 0.1 to 30.0% by weight of structural units originating from the monomer ethylene and 70.0 to 99.9% by weight of structural units originating from the monomer propylene.

6. A preparation as claimed in one or more of claims 1 to 5, wherein the homopolymer and/or copolymer waxes of the monomers ethylene and/or propylene have a ring/sphere dropping or softening point between 80 and 165°C and a melt viscosity, measured at a temperature of 170°C, between 20 and 40 000 mPa·s.

7. A preparation as claimed in one or more of claims 1 to 6, wherein the copolymer waxes of the monomers ethylene and propylene have a glass transition temperature of at most -20°C.

8. A preparation as claimed in one or more of claims 1 to 7, wherein the homopolymer and/or copolymer waxes of the monomers ethylene and/or propylene have a degree of crystallization of more than 55% and preferably between 60 and 90%.

9. A preparation as claimed in one or more of claims 1 to 8, wherein the homopolymer and/or copolymer waxes of the monomers ethylene and/or propylene have a degree of crystallization below 55% and preferably below 50%.

10. A preparation as claimed in one or more of claims 1 to 9, which comprises one or more homopolymer and/or copolymer waxes which are not polar modified, and additionally one or more homopolymer and/or copolymer waxes which are polar modified.

11. A preparation as claimed in one or more of claims 1 to 9, wherein the homopolymer and/or copolymer waxes are chosen from non-polar modified homopolymer and/or copolymer waxes.

12. A preparation as claimed in one or more of claims 1 to 9, wherein the homopolymer and/or copolymer waxes are chosen from polar modified homopolymer and/or copolymer waxes.

13. A preparation as claimed in one or more of claims 1 to 12, which is a pencil, cover stick, acne stick, lipstick, a make-up, a foundation, a face powder, blusher, a mascara, an eyeshadow, eyeliner, a peel cream, a hair wax, hair styling composition, styling fluid, a hair foam, a hair gel, hair spray, a mousse, a hair oil, an end fluid, a hair treatment, night cream, care cream, nutrient cream, perfume cream, body lotion, ointment, a lipcare composition, sunscreen composition, deodorant, antiperspirant, colored gel in the form of a pencil, such as, for example, a multiphase pencil, a stick, a paste, a powder, a cream, a cream foam, a lotion, a self-foaming, foam-like, after-foaming or foamable emulsion, a gel, roll-on preparation, a foam or a depilatory.

14. A preparation as claimed in claim 13, which is in the form of a stick or pencil.

15. A preparation as claimed in one or more of claims 1 to 14, which is in the form of an emulsion.

16. A preparation as claimed in claim 15, which is in the form of an oil-in-water emulsion.

17. A preparation as claimed in claim 16, which is in the form of an emulsion of the oil-in-water type, preferably cosmetic or dermatological emulsion of the oil-in-water type, and, based on the total weight of the preparation, comprises
a) up to 95% by weight, preferably 60 to 92% by weight, particularly preferably 70 to 90% by weight, especially preferably 75 to 85% by weight, of a water phase,
b) up to 40% by weight, preferably 1 to 40% by weight, particularly preferably 2 to 25% by weight, especially preferably 5 to 20% by weight, of an oil phase,
c) up to 15% by weight, preferably 0.5 to 12% by weight, particularly preferably 1 to 8% by weight, especially preferably 1 to 5% by weight, of one or more emulsifiers and
d) up to 5% by weight, preferably 0.01 to 5% by weight, particularly preferably 0.05 to 3% by weight, especially preferably 0.1 to 2% by weight, of homopolymer and/or copolymer wax.

18. A preparation as claimed in claim 16, which is in the form of a gel cream of the oil-in-water type, preferably cosmetic or dermatological gel cream of the oil-in-water type, and, based on the total weight of the preparation, comprises
a) up to 95% by weight, preferably 50 to 95% by weight, particularly preferably 70 to 90% by weight, especially preferably 75 to 85% by weight, of a water phase,
b) up to 30% by weight, preferably 1 to 30% by weight, particularly preferably 3 to 25% by weight, especially preferably 5 to 15% by weight, of an oil phase,
c) up to 5% by weight, preferably 0.5 to 5% by weight, particularly preferably 0.2 to 4% by weight, especially preferably 0.5 to 3% by weight, of one or more emulsifiers and
d) up to 5% by weight, preferably 0.01 to 5% by weight, particularly preferably 0.05 to 3% by weight, especially preferably 0.1 to 2% by weight, of homopolymer and/or copolymer wax.

19. A preparation as claimed in claim 15, which is in the form of a water-in-oil emulsion.

20. A preparation as claimed in claim 19, which is in the form of an emulsion of the water-in-oil type, preferably cosmetic or dermatological emulsion of the water-in-oil type, and, based on the total weight of the preparation, comprises
a) up to 95% by weight, preferably 40 to 95% by weight, particularly preferably 50 to 90% by weight, especially preferably 60 to 85% by weight, of a water phase,
b) up to 60% by weight, preferably 2 to 60% by weight, particularly preferably 5 to 40% by weight, especially preferably 10 to 30% by weight, of an oil phase,
c) up to 20% by weight, preferably 0.5 to 20% by weight, particularly preferably 1 to 15% by weight, especially preferably 4 to 12% by weight, of one or more emulsifiers and
d) up to 5% by weight, preferably 0.01 to 5% by weight, particularly preferably 0.05 to 3% by weight, especially preferably 0.1 to 2% by weight, of homopolymer and/or copolymer wax.

21. A preparation as claimed in one or more of claims 1 to 20, wherein the homopolymer and/or copolymer waxes are used in micronized form.

22. A preparation as claimed in one or more of claims 1 to 21, which is in the form of a dispersion and comprises
a) a carrier material, preferably one or more oil components and/or solvents,
b) one or more emulsifiers and
c) besides the homopolymer and/or copolymer waxes specified in claim 1, optionally one or more further waxes.

23. A preparation as claimed in one or more of claims 1 to 22, which is in the form of a decorative composition.

24. A preparation as claimed in one or more of claims 1 to 23, preferably in the form of a powder, compact, a paste, cream or a stick, which comprises one or more colorants, preferably chosen from color lakes, toners and pigments.

25. A preparation as claimed in one or more of claims 1 to 24, which comprises one or more UV photoprotective filters, is preferably a sunscreen composition, and particularly preferably is in the form of a spray, stick, a paste, a gel or a lotion.

26. A preparation as claimed in one or more of claims 1 to 25, which comprises one or more antioxidants.

27. A preparation as claimed in one or more of claims 1 to 22 and 24 to 26, preferably in the form of a spray, stick, a paste, a gel or a lotion, which is a deodorant or antiperspirant and comprises one or more substances chosen from antimicrobially effective substances, astringents and deodorizing substances.

28. A preparation as claimed in claim 27, which comprises one or more deodorizing substances and these are chosen from allantoin and bisabolol.

29. A preparation as claimed in one or more of claims 1 to 22 and 24 to 26, which is a peel.

30. A preparation as claimed in one or more of claims 1 to 26, which is a dental care composition.

31. A preparation as claimed in one or more of claims 1 to 22 and 24 to 26, which is a hair removal composition.

## Revendications

1. Préparation cosmétique, pharmaceutique ou dermatologique, contenant une ou plusieurs cires homopolymères et/ou copolymères à base des monomères éthylène et/ou propylène, **caractérisée en ce que** les cires homopolymères et copolymères ont une masse moléculaire moyenne en poids Mw inférieure ou égale à 25 000 g/mole, une masse moléculaire moyenne en nombre Mn inférieure ou égale à 15 000 g/mole, une distribution de masse moléculaire Mw/Mn dans la plage de 1,5 à 10, de préférence de 1,5 à 5, de façon particulièrement préférée de 1,5 à 3 et de façon tout particulièrement préférée de 2 à 2,5 et ont été obtenues par catalyse par des métallocènes, et dans laquelle les cires copolymères contiennent, par rapport au poids total des cires copolymères, 0,1 à 30,0 % en poids de motifs structuraux provenant de l'un des monomères et 70,0 à 99,9 % en poids de motifs structuraux provenant de l'autre monomère.

2. Préparation selon la revendication 1, contenant une ou plusieurs cires homopolymères à base du monomère éthylène, **caractérisée en ce que** les cires homopolymères ont une masse moléculaire moyenne en poids Mw inférieure ou égale à 25 000 g/mole, de préférence de 1 000 à 22 000 g/mole et de façon particulièrement préférée, de 4 000 à 20 000 g/mole, une masse moléculaire moyenne en nombre Mn inférieure ou égale à 15 000 g/mole, de préférence de 500 à 12 000 g/mole et de façon particulièrement préférée, de 1 000 à 5 000 g/mole et une distribution de masse moléculaire Mw/Mn dans la plage de 1,5 à 10, de préférence de 1,5 à 5, de façon particulièrement préférée de 1,5 à 3 et de façon tout particulièrement préférée de 2 à 2,5.

3. Préparation selon la revendication 1, contenant une ou plusieurs cires copolymères à base des monomères éthylène et propylène, **caractérisée en ce que** les cires copolymères ont une masse moléculaire moyenne en poids Mw inférieure ou égale à 25 000 g/mole, de préférence de 2 000 à 22 000 g/mole et de façon particulièrement préférée, de 4 000 à 20 000 g/mole, une masse moléculaire moyenne en nombre Mn inférieure ou égale à 15 000 g/mole, de préférence de 1 000 à 12 000 g/mole et de façon particulièrement préférée, de 2 000 à 10 000 g/mole et une distribution de masse moléculaire Mw/Mn dans la plage de 1,5 à 10, de préférence de 1,5 à 5, de façon particulièrement préférée de 1,5 à 3 et de façon tout particulièrement préférée de 2 à 2,5 et les cires copolymères contiennent, par rapport au poids total des cires copolymères, 70,0 à 99,9 % en poids de motifs structuraux provenant du monomère éthylène et 0,1 à 30,0 % en poids de motifs structuraux provenant du monomère propylène.

4. Préparation selon la revendication 1, contenant une ou plusieurs cires homopolymères à base du monomère propylène, **caractérisée en ce que** les cires homopolymères ont une masse moléculaire moyenne en poids Mw inférieure ou égale à 25 000 g/mole, de préférence de 2 000 à 22 000 g/mole et de façon particulièrement préférée, de 4 000 à 22 000 g/mole, une masse moléculaire moyenne en nombre Mn inférieure ou égale à 15 000 g/mole, de préférence de 1 000 à 12 000 g/mole et de façon particulièrement préférée, de 2 000 à 10 000 g/mole, une distribution de masse moléculaire Mw/Mn dans la plage de 1,5 à 10, de préférence de 1,5 à 5, de façon particulièrement préférée de 1,5 à 3 et de façon tout particulièrement préférée de 2 à 2,5, et un indice isotactique de plus de 70 %, de préférence de 75 à 95 % et de façon particulièrement préférée de 80 à 90 %.

5. Préparation selon la revendication 1, contenant une ou plusieurs cires copolymères à base des monomères éthylène et propylène, **caractérisée en ce que** les cires copolymères ont une masse moléculaire moyenne en poids Mw inférieure ou égale à 25 000 g/mole, de préférence de 2 000 à 22 000 g/mole et de façon particulièrement préférée, de 4 000 à 20 000 g/mole, une masse moléculaire moyenne en nombre Mn inférieure ou égale à 15 000 g/mole, de préférence de 1 000 à 12 000 g/mole et de façon particulièrement préférée, de 2 000 à 10 000 g/mole, une distribution de masse moléculaire Mw/Mn dans la plage de 1,5 à 10, de préférence de 1,5 à 5, de façon particulièrement préférée de 1,5 à 3 et de façon tout particulièrement préférée de 2 à 2,5 et les cires copolymères contiennent, par rapport au poids total des cires copolymères, 0,1 à 30,0 % en poids de motifs structuraux provenant du monomère éthylène et 70,0 à 99,9 % en poids de motifs structuraux provenant du monomère propylène.

6. Préparation selon une ou plusieurs des revendications 1 à 5, **caractérisée en ce que** les cires homopolymères et/ou copolymères à base des monomères éthylène et/ou propylène ont un point de goutte ou de ramollissement à l'anneau/bille compris entre 80 et 165 °C et une viscosité à chaud, mesurée à une température de 170 °C, comprise entre 20 et 40 000 mPa.s.

7. Préparation selon une ou plusieurs des revendications 1 à 6, **caractérisée en ce que** les cires copolymères à base des monomères éthylène et propylène présentent une température de transition vitreuse d'au maximum -20 °C.

8. Préparation selon une ou plusieurs des revendications 1 à 7, **caractérisée en ce que** les cires homopolymères et/ou copolymères à base des monomères éthylène et/ou propylène présentent un degré de cristallisation de plus de 55 % et de préférence compris entre 60 et 90 %.

9. Préparation selon une ou plusieurs des revendications 1 à 8, **caractérisée en ce que** les cires homopolymères et/ou copolymères à base des monomères éthylène et/ou propylène présentent un degré de cristallisation inférieur à 55 % et de préférence inférieur à 50 %.

10. Préparation selon une ou plusieurs des revendications 1 à 9, **caractérisée en ce qu'**elle contient une ou plusieurs cires homopolymères et/ou copolymères qui ne pas à modification polaire, et en outre une ou plusieurs cires homopolymères et/ou copolymères qui sont à modification polaire.

11. Préparation selon une ou plusieurs des revendications 1 à 9, **caractérisée en ce que** les cires homopolymères et/ou copolymères sont choisies parmi des cires homopolymères et/ou copolymères qui ne sont pas à modification polaire.

12. Préparation selon une ou plusieurs des revendications 1 à 9, **caractérisée en ce que** les cires homopolymères et/ou copolymères sont choisies parmi des cires homopolymères et/ou copolymères à modification polaire.

13. Préparation selon une ou plusieurs des revendications 1 à 12, **caractérisée en ce qu'**il s'agit d'un bâton, d'un crayon couvrant, d'un bâton anti-acnéique, d'un bâton pour les lèvres, d'un produit de maquillage, d'un fond de teint, d'une poudre pour le visage, d'un rouge à joues, d'un mascara, d'une ombre à paupières, d'un crayon ligneur, d'une crème exfoliante, d'une cire capillaire, d'une produit de coiffage, d'un liquide coiffant, d'une mousse capillaire, d'un gel capillaire, d'une laque pour cheveux, d'une mousse, d'une huile capillaire, d'un liquide pour pointes, d'une lotion de soin capillaire, d'une crème de nuit, d'une crème de soin, d'une crème nutritive, d'une crème parfumée, d'une lotion pour bébé, d'une pommade, d'un produit pour le soin des lèvres, d'un produit de protection solaire, d'un déodorant, d'un antisudoral, d'un gel coloré sous forme d'un bâton, comme par exemple d'un bâton multiphase, d'un bâtonnet (stick), d'une pâte, d'une poudre, d'une crème, d'une mousse-crème, d'une lotion, d'une émulsion automoussante, sous forme de mousse, post-moussante ou apte à être transformée en mousse, d'un gel, d'une préparation à appliquer à l'applicateur à bille, d'une mousse ou d'un produit épilatoire.

14. Préparation la revendication 13, **caractérisée en ce qu'**elle se trouve sous forme d'un bâtonnet ou d'un bâton.

15. Préparation selon une ou plusieurs des revendications 1 à 14, **caractérisée en ce qu'**elle se trouve sous forme d'une émulsion.

16. Préparation la revendication 15, **caractérisée en ce qu'**elle se trouve sous forme d'une émulsion huile-dans-eau.

17. Préparation la revendication 16, **caractérisée en ce qu'**elle se trouve sous forme d'une émulsion du type huile-dans-eau, de préférence sous forme d'une émulsion cosmétique ou dermatologique du type huile-dans-eau, et contient, par rapport au poids total de la préparation
a) jusqu'à 95 % en poids, de préférence de 60 à 92 % en poids, de façon particulièrement préférée de 70 à 90 % en poids, de façon tout particulièrement préférée de 75 à 85 % en poids d'une phase aqueuse,
b) jusqu'à 40 % en poids, de préférence de 1 à 40 % en poids, de façon particulièrement préférée de 2 à 25 % en poids, de façon tout particulièrement préférée de 5 à 20 % en poids d'une phase huileuse,
c) jusqu'à 15 % en poids, de préférence de 0,5 à 12 % en poids, de façon particulièrement préférée de 1 à 8 % en poids, de façon tout particulièrement préférée de 1 à 5 % en poids d'un ou plusieurs émulsifiants et
d) jusqu'à 5 % en poids, de préférence de 0,01 à 5 % en poids, de façon particulièrement préférée de 0,05 à 3 % en poids, de façon tout particulièrement préférée de 0,1 à 2 % en poids de cire homopolymère et/ou copolymère.

18. Préparation la revendication 16, **caractérisée en ce qu'**elle se trouve sous forme de crème-gel du type huile-dans-eau, de préférence sous forme de crème-gel cosmétique ou dermatologique du type huile-dans-eau, et contient, par rapport au poids total de la préparation
a) jusqu'à 95 % en poids, de préférence de 50 à 95 % en poids, de façon particulièrement préférée de 70 à 90 % en poids, de façon tout particulièrement préférée de 75 à 85 % en poids d'une phase aqueuse,
b) jusqu'à 30 % en poids, de préférence de 1 à 30 % en poids, de façon particulièrement préférée de 3 à 25 % en poids, de façon tout particulièrement préférée de 5 à 15 % en poids d'une phase huileuse,
c) jusqu'à 5 % en poids, de préférence de 0,5 à 5 % en poids, de façon particulièrement préférée de 0,2 à 4 % en poids, de façon tout particulièrement préférée de 0,5 à 3 % en poids d'un ou plusieurs émulsifiants et
d) jusqu'à 5 % en poids, de préférence de 0,01 à 5 % en poids, de façon particulièrement préférée de 0,05 à 3 % en poids, de façon tout particulièrement préférée de 0,1 à 2 % en poids de cire homopolymère et/ou copolymère.

19. Préparation la revendication 15, **caractérisée en ce qu'**elle se trouve sous forme d'une émulsion eau-dans-huile.

20. Préparation la revendication 19, **caractérisée en ce qu'**elle se trouve sous forme d'une émulsion du type eau-dans-huile, de préférence sous forme d'une émulsion cosmétique ou dermatologique du type eau-dans-huile, et contient, par rapport au poids total de la préparation
a) jusqu'à 95 % en poids, de préférence de 40 à 95 % en poids, de façon particulièrement préférée de 50 à 90 % en poids, de façon tout particulièrement préférée de 60 à 85 % en poids d'une phase aqueuse,
b) jusqu'à 60 % en poids, de préférence de 2 à 60 % en poids, de façon particulièrement préférée de 5 à 40 % en poids, de façon tout particulièrement préférée de 10 à 30 % en poids d'une phase huileuse,
c) jusqu'à 20 % en poids, de préférence de 0,5 à 20 % en poids, de façon particulièrement préférée de 1 à 15 % en poids, de façon tout particulièrement préférée de 4 à 12 % en poids d'un ou plusieurs émulsifiants et
d) jusqu'à 5 % en poids, de préférence de 0,01 à 5 % en poids, de façon particulièrement préférée de 0,05 à 3 % en poids, de façon tout particulièrement préférée de 0,1 à 2 % en poids de cire homopolymère et/ou copolymère.

21. Préparation selon une ou plusieurs des revendications 1 à 20, **caractérisée en ce que** les cires homopolymères et/ou copolymères sont utilisées sous forme micronisée.

22. Préparation selon une ou plusieurs des revendications 1 à 21, **caractérisée en ce qu'**elle se trouve sous forme d'une dispersion et contient
a) une matière de support, de préférence un ou plusieurs composants huileux et/ou solvants,
b) un ou plusieurs émulsifiants et
c) en plus des cires homopolymères et/ou copolymères mentionnées dans la revendication 1, éventuellement une ou plusieurs autres cires.

23. Préparation selon une ou plusieurs des revendications 1 à 22, **caractérisée en ce qu'**elle est présente sous forme d'un produit de maquillage.

24. Préparation selon une ou plusieurs des revendications 1 à 23, de préférence sous forme d'une poudre, d'une poudre pressée, d'une pâte, d'une crème ou d'un bâtonnet, **caractérisée en ce qu'**elle contient un ou plusieurs agents colorants choisis de préférence parmi des vernis colorés, des toners et des pigments.

25. Préparation selon une ou plusieurs des revendications 1 à 24, **caractérisée en ce qu'**elle contient un ou plusieurs filtres anti-UV, de préférence est un produit antisolaire, et se trouve de façon particulièrement préférée sous forme d'un produit à pulvériser en aérosol, d'un bâtonnet, d'une pâte, d'un gel ou d'une lotion.

26. Préparation selon une ou plusieurs des revendications 1 à 25, **caractérisée en ce qu'**elle contient un ou plusieurs antioxydants.

27. Préparation selon une ou plusieurs des revendications 1 à 22 et 24 à 26, de préférence sous forme d'un produit à pulvériser en aérosol, d'un bâtonnet, d'une pâte, d'un gel ou d'une lotion, **caractérisée en ce qu'**elle est un déodorant ou antisudoral et contient une ou plusieurs substances choisies parmi des substances à activité antimicrobienne, des astringents et des substances déodorantes.

28. Préparation selon la revendication 27,
**caractérisée en ce qu'**elle contient une ou plusieurs substances déodorantes et celles-ci sont choisies parmi l'allantoïne et le bisabolol.

29. Préparation selon une ou plusieurs des revendications 1 à 22 et 24 à 26, **caractérisée en ce qu'**elle est un exfoliant.

30. Préparation selon une ou plusieurs des revendications 1 à 26, **caractérisée en ce qu'**elle est un produit pour le soin des dents.

31. Préparation selon une ou plusieurs des revendications 1 à 22 et 24 à 26, **caractérisée en ce qu'**elle est un produit épilatoire.
